# EUROPEAN PATENT APPLICATION

(11) **EP 3 124 466 A1**
(43) Date of publication of application: **01.02.2017**
(21) Application number: 15770047.7
(22) Date of filing: 24.03.2015
(51) Int. Cl.: C07C 211/54, C07C 209/10, C09K 11/06, H01L 51/50, C07B 61/00

(54) **TRIARYLAMINE DERIVATIVE AND USE OF SAME**

(30) Priority: 27.03.2014 JP 2014065217
(71) Applicant: Nissan Chemical Industries, Ltd., Chiyoda-ku Tokyo 101-0054 (JP)
(72) Inventor: NAKAZAWA, Taichi, Funabashi-shi Chiba 274-0052 (JP); KOGA, Haruka, Funabashi-shi Chiba 274-0052 (JP)
(74) Representative: Bailey, Sam Rogerson
(86) International application number: PCT/JP2015/058867
(87) International publication number: WO 2015/146965

(57) **Abstract**

Provided is a triarylamine derivative represented by formula (1). 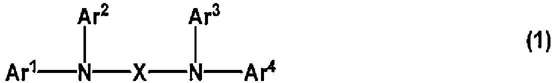 [In the formula, Ar1-Ar4 each independently represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group, and X represents a divalent group represented by formula (2). 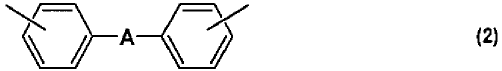 (In the formula, A represents a C1-C6 fluoroalkanediyl group.)]

## Description

### TECHNICAL FIELD

The present invention relates to triarylamine derivative and application thereof.

### BACKGROUND ART

Organic electroluminescence (EL) elements are expected to find practical use in such fields as display and illumination. Efforts toward developments relating to their structure and materials are being made to achieve such objects as low-voltage driving, high luminance, and extended life.

Any organic EL element is provided with a plurality of functional thin films including a hole injection layer and a hole transporting layer. These layers transport charges from an anode to an emitting layer. They play an important role for the organic EL element to meet requirements for low-voltage driving and high luminance.

Such layers are prepared mostly by dry process typified by vapor deposition or wet process typified by spin coating. The wet process has an advantage over the dry process in its ability to efficiently form flat thin film over a large area. Therefore, the wet process prevails in the field of display.

Under these circumstances, there has been a constant demand for improvement in a wet process material used for forming the hole injection layer and hole transporting layer. One way to meet this demand is by incorporating a silane compound into the composition including a conductive polymer, and it has been reported that the thus obtained composition gives rise to a thin film which improves the life and luminance characteristics of the organic EL element (see Patent Documents 1 and 2, for example). However, very little has been reported so far about the triarylamine derivatives used for the above-mentioned object.

### PRIOR ART DOCUMENTS

### PATENT DOCUMENTS

Patent Document 1: JP-A 2003-045667
Patent Document 2: JP-A 2007-169593

### SUMMARY OF THE INVENTION

### PROBLEMS TO BE SOLVED BY THE INVENTION

The present invention has been made in view of the above circumstances. An object of the present invention is to provide triarylamine derivatives which exhibit good solubility in an organic solvent and readily dissolve together with a charge transporting substance in the organic solvent, giving rise to a varnish, which can be made into a thin film that functions as a hole injection layer. The organic EL element with this hole injection layer possesses an outstanding luminance characteristics.

### MEANS FOR SOLVING THE PROBLEMS

The present inventors carried out extensive investigations to achieve the above objects. As a result, the inventors have found that triarylamine derivatives having a structure that two triarylamine skeletons bound to a fluoroalkanediyl group have good solubility in an organic solvent. The inventors also have found that when dissolved in an organic solvent together with a charge transporting substance, the triarylamine derivative defined above gives rise to a varnish, which can be made into a thin film having high charge transportability, and the thus obtained thin film can be used as the hole injection layer for an organic EL element having high luminance. Thus, the present invention has been completed.

Thus, the present invention provides a triarylamine derivative and application thereof as defined below.
1. A triarylamine derivative represented by formula (1): 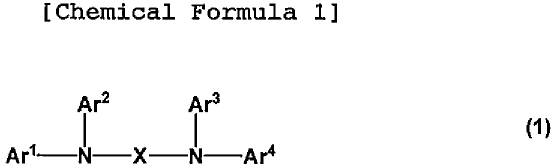 [wherein, Ar¹ to Ar⁴ each independently represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group; and X' represents a divalent group represented by formula (2): 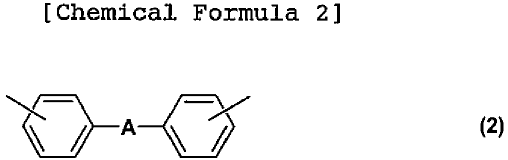 (wherein A represents a fluoroalkanediyl group having 1 to 6 carbon atoms)].
2. The triarylamine derivative of 1 above, wherein Ar¹ to Ar⁴ simultaneously represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group.
3. The triarylamine derivative of 1 or 2 above, wherein X represents a group represented by formulae (X1) to (X3): 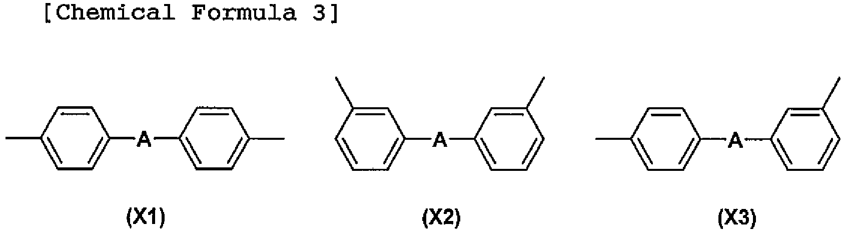 (wherein A is defined as above).
4. The triarylamine derivative of 3 above, wherein X represents a group represented by formula (X1).
5. The triarylamine derivative of any one of 1 to 4 above, wherein A represents a perfluoromethanediyl group, perfluoroethane-1,2-diyl group, perfluoropropane-1,3-diyl group, perfluoropropane-2,2-diyl group, perfluorobutane-1,4-diyl group, perfluoropentane-1,5-diyl group, or perfluorohexane-1,6-diyl group.
6. The triarylamine derivative of 5 above, wherein A represents a perfluoropropane-2,2-diyl group.
7. A charge transporting varnish including:
   the triarylamine derivative of any one of 1 to 6 above;
   a charge transporting substance; and
   an organic solvent.
8. The charge transporting varnish of 7 above, further including a dopant.
9. The charge transporting varnish of 8 above, wherein the dopant includes a heteropoly acid.
10. A charge transporting thin film produced by use of the charge transporting varnish of any one of 7 to 9 above.
11. An organic EL element including the charge transporting thin film of 10 above.
12. A method of producing a charge transporting thin film, the method including:
   coating a substrate with the charge transporting varnish of any one of 7 to 9 above; and
   evaporating a solvent.
13. A method of producing the triarylamine derivative of 1 above, the method including
   reacting in the presence of catalyst an amine compound represented by formula (3) with compounds represented by formulae (4) to (7): 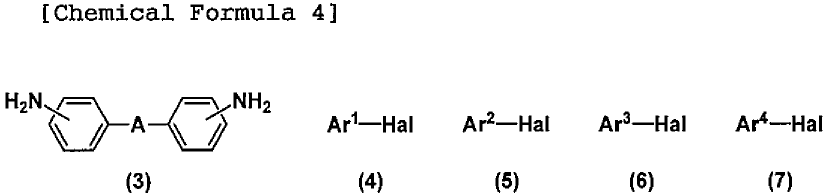 (wherein Ar¹ to Ar⁴ and A are defined as above, and Hal represents a halogen atom or pseudohalogen group).
14. A method of producing the triarylamine derivative of 1 above, the method including
   reacting in the presence of catalyst a compound represented by formula (3') with amine compounds represented by formulae (4') and (5'): 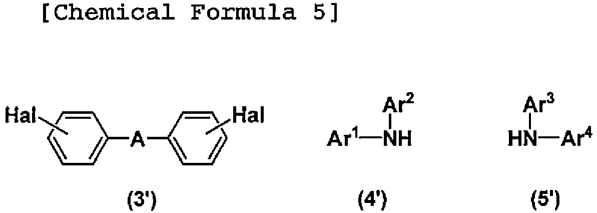 (wherein Ar¹ to Ar⁴ and A are defined as above, and Hal represents a halogen atom or pseudohalogen group).

### ADVANTAGEOUS EFFECTS OF THE INVENTION

The triarylamine derivative according to the present invention is readily soluble in an organic solvent. When dissolved in an organic solvent together with a charge transporting substance, it easily gives rise to a charge transporting varnish.

This charge transporting varnish according to the present invention can be made into a thin film which exhibits a high charge transportability, so that the thin film finds use for organic EL elements and other various electronic devices. The triarylamine derivative according to the present invention functions as an agent which allows to improve hole transporting to the emitting layer and hole transporting layer. Therefore, when used as a hole injection layer of an organic EL element, this thin film contributes to organic EL elements superior in luminance characteristics.

Moreover, the charge transporting varnish according to the present invention consistently gives rise to thin film superior in charge transportability even when applied by wet processes, such as spin coating and slit coating, which are suitable for large-area coating. Because of this performance, the charge transporting varnish is adaptable to the recent highly developed organic EL elements.

In addition, the triarylamine derivative according to the present invention is expected to find use as a raw material for the charge transporting polymer compound.

### EMBODIMENT FOR CARRYING OUT THE INVENTION

### [Triarylamine derivative]

The triarylamine derivative according to the present invention is represented by formula (1):

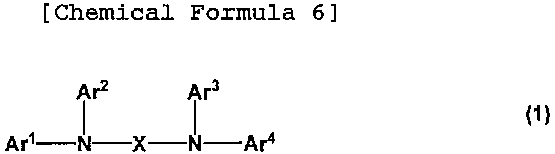

In formula (1), Ar¹ to Ar⁴ each independently represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group.

Also, X represents a divalent group represented by formula (2):

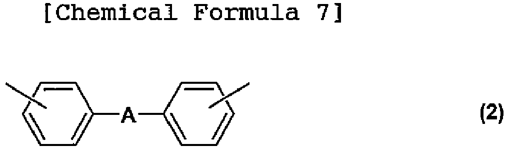

In formula (2), A represents a fluoroalkanediyl group having 1 to 6 carbon atoms. The fluoroalkanediyl group is not specifically restricted so long as hydrogen atoms bonded to the carbon atom of the alkanediyl group are substituted partly or entirely with fluorine atoms.

Typical examples of the fluoroalkanediyl group include monofluoromethanediyl group, perfluoromethanediyl group, 2,2,2-trifluoroethane-1,1-diyl group, perfluoroethane-1,1-diyl group, perfluoroethane-1,2-diyl group, 3-fluoropro-pane-1,2-diyl group, 3,3,3-trifluoropropane-1,1-diyl group, 1,1-difluoropropane-1,3-diyl group, perfluoropropane-1,1-diyl group, perfluoropropane-1,2-diyl group, perfluoropropane-1,3-diyl group, perfluoropropane-2,2-diyl group, 2-methyl-2-fluoropropane-1,3-diyl group, 3,4,4-trifluorobut-ane-1,2-diyl group, 4,4,4-trifluorobutane-1,3-diyl group, 2,2,3,3-tetrafluorobutane-1,4-diyl group, perfluorobutane-1,1-diyl group, perfluorobutane-1,2-diyl group, perfluoro-butane-1,3-diyl group, perfluoro-butane-1,4-diyl group, 1-fluoropentane-1,1-diyl group, 4,5,5-trifluoropentane-1,5-diyl group, 2,2,3,3,4,4-hexafluoropentane-1,5-diyl group, perfluoropentane-1,1-diyl group, perfluoropentane-1,2-diyl group, perfluoropentane-1,3-diyl group, perfluoropentane-1,4-diyl group, perfluoropentane-1,5-diyl group, 2,2,3,3,4,4,5,5-octafluorohexane-1,6-diyl group, perfluoro-haxane-1,1-diyl group, perfluoro-haxane-1,2-diyl group, perfluoro-haxane-1,3-diyl group, perfluoro-haxane-1,4-diyl group, perfluoro-haxane-1,5-diyl group, and perfluoro-haxane-1,6-diyl group.

Preferable among these examples are perfluoromethanediyl group, perfluoroethane-1,2-diyl group, perfluoropropane-1,3-diyl group, perfluoropropane-2,2-diyl group, perfluorobutane-1,4-diyl group, perfluoropentane-1,5-diyl group, and perfluorohexane-1,6-diyl group.

Most desirable among these examples, as A, is perfluoropropane-2,2-diyl group from the standpoint of balance of its readily available raw material compound, its ability to give the triarylamine derivative highly soluble in organic solvents, and its ability to give the triarylamine derivative which contributes to the high charge transportability.

To be more specific, X is any one group represented by formulae (X1) to (X6): 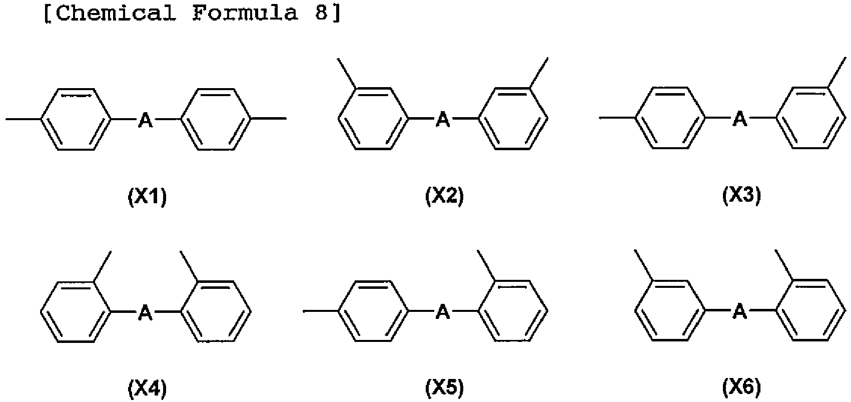 (wherein A is defined as above).

Preferable among these examples are those represented by formula (X1), (X2), or (X3), from the standpoint of readily available raw material compound.

Most desirable among these examples, as X, is the group represented by formula (X1) from the standpoint of balance of its readily available raw material compound, its ability to give the triarylamine derivative highly soluble in organic solvents, and its ability to give the triarylamine derivative which contributes to the high charge transportability.

Listed in the following tables are typical (but unrestricted) examples of the triarylamine derivative according to the present invention. Symbols shown in the tables are defined as follows. "Ph" is phenyl group, "1-NAP" is 1-naphtyl group, "2-NAP" is 2-naphtyl group, "1-ANT" is 1-anthryl group, "2-ANT" is 2-anthryl group, "1-PHE" is 1-phenanthryl group, "2-PHE" is 2-phenanthryl group, "3-PHE" is 3-phenanthryl group, "4-PHE" is 4-phenanthryl group, and "9-PHE" is 9-phenanthryl group. In addition, each example in the tables of "Ar¹ to Ar⁴," "X," and "A" represents the substituent group in formula (1) for the exemplified compound in the columns. For instance, in the following tables, the compound specified by (1-1) is represented below:

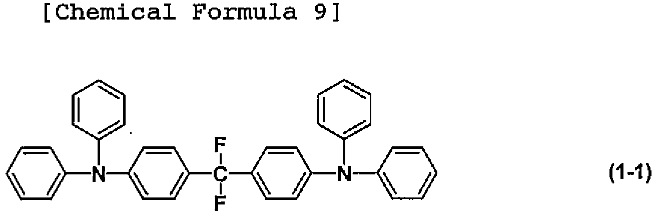

**[Table 1]**

| Compound | Ar¹ | Ar² | Ar³ | Ar⁴ | X | A |
|---|---|---|---|---|---|---|
| (1-1) | Ph | Ph | Ph | Ph | (X1) | Perfluoromethanediyl group |
| (1-2) | 1-NAP | 1-NAP | 1-NAP | 1-NAP | (X1) | Perfluoromethanediyl group |
| (1-3) | 2-NAP | 2-NAP | 2-NAP | 2-NAP | (X1) | Perfluoromethanediyl group |
| (1-4) | 1-ANT | 1-ANT | 1-ANT | 1-ANT | (X1) | Perfluoromethanediyl group |
| (1-5) | 2-ANT | 2-ANT | 2-ANT | 2-ANT | (X1) | Perfluoromethanediyl group |
| (1-6) | 1-PHE | 1-PHE | 1-PHE | 1-PHE | (X1) | Perfluoromethanediyl group |
| (1-7) | 2-PHE | 2-PHE | 2-PHE | 2-PHE | (X1) | Perfluoromethanediyl group |
| (1-8) | 3-PHE | 3-PHE | 3-PHE | 3-PHE | (X1) | Perfluoromethanediyl group |
| (1-9) | 4-PHE | 4-PHE | 4-PHE | 4-PHE | (X1) | Perfluoromethanediyl group |
| (1-10) | 9-PHE | 9-PHE | 9-PHE | 9-PHE | (X1) | Perfluoromethanediyl group |

**[Table 2]**

| Compound | Ar¹ | Ar² | Ar³ | Ar⁴ | X | A |
|---|---|---|---|---|---|---|
| (1-11) | Ph | Ph | Ph | Ph | (X1) | Perfluoroethane-1,2-diyl group |
| (1-12) | 1-NAP | 1-NAP | 1-NAP | 1-NAP | (X1) | Perfluoroethane-1,2-diyl group |
| (1-13) | 2-NAP | 2-NAP | 2-NAP | 2-NAP | (X1) | Perfluoroethane-1,2-diyl group |
| (1-14) | 1-ANT | 1-ANT | 1-ANT | 1-ANT | (X1) | Perfluoroethane-1,2-diyl group |
| (1-15) | 2-ANT | 2-ANT | 2-ANT | 2-ANT | (X1) | Perfluoroethane-1,2-diyl group |
| (1-16) | 1-PHE | 1-PHE | 1-PHE | 1-PHE | (X1) | Perfluoroethane-1,2-diyl group |
| (1-17) | 2-PHE | 2-PHE | 2-PHE | 2-PHE | (X1) | Perfluoroethane-1,2-diyl group |
| (1-18) | 3-PHE | 3-PHE | 3-PHE | 3-PHE | (X1) | Perfluoroethane-1,2-diyl group |
| (1-19) | 4-PHE | 4-PHE | 4-PHE | 4-PHE | (X1) | Perfluoroethane-1,2-diyl group |
| (1-20) | 9-PHE | 9-PHE | 9-PHE | 9-PHE | (X1) | Perfluoroethane-1,2-diyl group |

**[Table 3]**

| Compound | Ar¹ | Ar² | Ar³ | Ar⁴ | X | A |
|---|---|---|---|---|---|---|
| (1-21) | Ph | Ph | Ph | Ph | (X1) | Perfluoropropane-1,3-diyl group |
| (1-22) | 1-NAP | 1-NAP | 1-NAP | 1-NAP | (X1) | Perfluoropropane-1,3-diyl group |
| (1-23) | 2-NAP | 2-NAP | 2-NAP | 2-NAP | (X1) | Perfluoropropane-1,3-diyl group |
| (1-24) | 1-ANT | 1-ANT | 1-ANT | 1-ANT | (X1) | Perfluoropropane-1,3-diyl group |
| (1-25) | 2-ANT | 2-ANT | 2-ANT | 2-ANT | (X1) | Perfluoropropane-1,3-diyl group |
| (1-26) | 1-PHE | 1-PHE | 1-PHE | 1-PHE | (X1) | Perfluoropropane-1,3-diyl group |
| (1-27) | 2-PHE | 2-PHE | 2-PHE | 2-PHE | (X1) | Perfluoropropane-1,3-diyl group |
| (1-28) | 3-PHE | 3-PHE | 3-PHE | 3-PHE | (X1) | Perfluoropropane-1,3-diyl group |
| (1-29) | 4-PHE | 4-PHE | 4-PHE | 4-PHE | (X1) | Perfluoropropane-1,3-diyl group |
| (1-30) | 9-PHE | 9-PHE | 9-PHE | 9-PHE | (X1) | Perfluoropropane-1,3-diyl group |

**[Table 4]**

| Compound | Ar¹ | Ar² | Ar³ | Ar⁴ | X | A |
|---|---|---|---|---|---|---|
| (1-31) | Ph | Ph | Ph | Ph | (X1) | Perfluoropropane-2,2-diyl group |
| (1-32) | 1-NAP | 1-NAP | 1-NAP | 1-NAP | (X1) | Perfluoropropane-2,2-diyl group |
| (1-33) | 2-NAP | 2-NAP | 2-NAP | 2-NAP | (X1) | Perfluoropropane-2,2-diyl group |
| (1-34) | 1-ANT | 1-ANT | 1-ANT | 1-ANT | (X1) | Perfluoropropane-2,2-diyl group |
| (1-35) | 2-ANT | 2-ANT | 2-ANT | 2-ANT | (X1) | Perfluoropropane-2,2-diyl group |
| (1-36) | 1-PHE | 1-PHE | 1-PHE | 1-PHE | (X1) | Perfluoropropane-2,2-diyl group |
| (1-37) | 2-PHE | 2-PHE | 2-PHE | 2-PHE | (X1) | Perfluoropropane-2,2-diyl group |
| (1-38) | 3-PHE | 3-PHE | 3-PHE | 3-PHE | (X1) | Perfluoropropane-2,2-diyl group |
| (1-39) | 4-PHE | 4-PRE | 4-PHE | 4-PHE | (X1) | Perfluoropropane-2,2-diyl group |
| (1-40) | 9-PHE | 9-PHE | 9-PHE | 9-PHE | (X1) | Perfluoropropane-2,2-diyl group |

**[Table 5]**

| Compound | Ar¹ | Ar² | Ar³ | Ar⁴ | X | A |
|---|---|---|---|---|---|---|
| (1-41) | Ph | Ph | Ph | Ph | (X1) | Perfluorobutane-1,4-diyl group |
| (1-42) | 1-NAP | 1-NAP | 1-NAP | 1-NAP | (X1) | Perfluorobutane-1,4-diyl group |
| (1-43) | 2-NAP | 2-NAP | 2-NAP | 2-NAP | (X1) | Perfluorobutane-1,4-diyl group |
| (1-44) | 1-ANT | 1-ANT | 1-ANT | 1-ANT | (X1) | Perfluorobutane-1,4-diyl group |
| (1-45) | 2-ANT | 2-ANT | 2-ANT | 2-ANT | (X1) | Perfluorobutane-1,4-diyl group |
| (1-46) | 1-PHE | 1-PHE | 1-PHE | 1-PHE | (X1) | Perfluorobutane-1,4-diyl group |
| (1-47) | 2-PHE | 2-PHE | 2-PHE | 2-PHE | (X1) | Perfluorobutane-1,4-diyl group |
| (1-48) | 3-PHE | 3-PHE | 3-PHE | 3-PHE | (X1) | Perfluorobutane-1,4-diyl group |
| (1-49) | 4-PHE | 4-PHE | 4-PHE | 4-PHE | (X1) | Perfluorobutane-1,4-diyl group |
| (1-50) | 9-PHE | 9-PHE | 9-PHE | 9-PHE | (X1) | Perfluorobutane-1,4-diyl group |

**[Table 6]**

| Compound | Ar¹ | Ar² | Ar³ | Ar⁴ | X | A |
|---|---|---|---|---|---|---|
| (1-51) | Ph | Ph | Ph | Ph | (X1) | Perfluoropentane-1,5-diyl group |
| (1-52) | 1-NAP | 1-NAP | 1-NAP | 1-NAP | (X1) | Perfluoropentane-1,5-diyl group |
| (1-53) | 2-NAP | 2-NAP | 2-NAP | 2-NAP | (X1) | Perfluoropentane-1,5-diyl group |
| (1-54) | 1-ANT | 1-ANT | 1-ANT | 1-ANT | (X1) | Perfluoropentane-1,5-diyl group |
| (1-55) | 2-ANT | 2-ANT | 2-ANT | 2-ANT | (X1) | Perfluoropentane-1,5-diyl group |
| (1-56) | 1-PHE | 1-PHE | 1-PHE | 1-PHE | (X1) | Perfluoropentane-1,5-diyl group |
| (1-57) | 2-PHE | 2-PHE | 2-PHE | 2-PHE | (X1) | Perfluoropentane-1,5-diyl group |
| (1-58) | 3-PHE | 3-PHE | 3-PHE | 3-PHE | (X1) | Perfluoropentane-1,5-diyl group |
| (1-59) | 4-PHE | 4-PHE | 4-PHE | 4-PHE | (X1) | Perfluoropentane-1,5-diyl group |
| (1-60) | 9-PHE | 9-PHE | 9-PHE | 9-PHE | (X1) | Perfluoropentane-1,5-diyl group |

**[Table 7]**

| Compound | Ar¹ | Ar² | Ar³ | Ar⁴ | X | A |
|---|---|---|---|---|---|---|
| (1-61) | Ph | Ph | Ph | Ph | (X1) | Perfluorohexane-1,6-diyl group |
| (1-62) | 1-NAP | 1-NAP | 1-NAP | 1-NAP | (X1) | Perfluorohexane-1,6-diyl group |
| (1-63) | 2-NAP | 2-NAP | 2-NAP | 2-NAP | (X1) | Perfluorohexane-1,6-diyl group |
| (1-64) | 1-ANT | 1-ANT | 1-ANT | 1-ANT | (X1) | Perfluorohexane-1,6-diyl group |
| (1-65) | 2-ANT | 2-ANT | 2-ANT | 2-ANT | (X1) | Perfluorohexane-1,6-diyl group |
| (1-66) | 1-PHE | 1-PHE | 1-PHE | 1-PHE | (X1) | Perfluorohexane-1,6-diyl group |
| (1-67) | 2-PHE | 2-PHE | 2-PHE | 2-PHE | (X1) | Perfluorohexane-1,6-diyl group |
| (1-68) | 3-PHE | 3-PHE | 3-PHE | 3-PHE | (X1) | Perfluorohexane-1,6-diyl group |
| (1-69) | 4-PHE | 4-PHE | 4-PHE | 4-PHE | (X1) | Perfluorohexane-1,6-diyl group |
| (1-70) | 9-PHE | 9-PHE | 9-PHE | 9-PHE | (X1) | Perfluorohexane-1,6-diyl group |

**[Table 8]**

| Compound | Ar¹ | Ar² | Ar³ | Ar⁴ | X | A |
|---|---|---|---|---|---|---|
| (1-71) | Ph | Ph | Ph | Ph | (X2) | Perfluoromethanediyl group |
| (1-72) | 1-NAP | 1-NAP | 1-NAP | 1-NAP | (X2) | Perfluoromethanediyl group |
| (1-73) | 2-NAP | 2-NAP | 2-NAP | 2-NAP | (X2) | Perfluoromethanediyl group |
| (1-74) | 1-ANT | 1-ANT | 1-ANT | 1-ANT | (X2) | Perfluoromethanediyl group |
| (1-75) | 2-ANT | 2-ANT | 2-ANT | 2-ANT | (X2) | Perfluoromethanediyl group |
| (1-76) | 1-PHE | 1-PHE | 1-PHE | 1-PHE | (X2) | Perfluoromethanediyl group |
| (1-77) | 2-PHE | 2-PHE | 2-PHE | 2-PHE | (X2) | Perfluoromethanediyl group |
| (1-78) | 3-PHE | 3-PHE | 3-PHE | 3-PHE | (X2) | Perfluoromethanediyl group |
| (1-79) | 4-PHE | 4-PHE | 4-PHE | 4-PHE | (X2) | Perfluoromethanediyl group |
| (1-80) | 9-PHE | 9-PHE | 9-PHE | 9-PHE | (X2) | Perfluoromethanediyl group |

**[Table 9]**

| Compound | Ar¹ | Ar² | Ar³ | Ar⁴ | X | A |
|---|---|---|---|---|---|---|
| (1-81) | Ph | Ph | Ph | Ph | (X2) | Perfluoroethane-1,2-diyl group |
| (1-82) | 1-NAP | 1-NAP | 1-NAP | 1-NAP | (X2) | Perfluoroethane-1,2-diyl group |
| (1-83) | 2-NAP | 2-NAP | 2-NAP | 2-NAP | (X2) | Perfluoroethane-1,2-diyl group |
| (1-84) | 1-ANT | 1-ANT | 1-ANT | 1-ANT | (X2) | Perfluoroethane-1,2-diyl group |
| (1-85) | 2-ANT | 2-ANT | 2-ANT | 2-ANT | (X2) | Perfluoroethane-1,2-diyl group |
| (1-86) | 1-PHE | 1-PHE | 1-PHE | 1-PHE | (X2) | Perfluoroethane-1,2-diyl group |
| (1-87) | 2-PHE | 2-PHE | 2-PHE | 2-PHE | (X2) | Perfluoroethane-1,2-diyl group |
| (1-88) | 3-PHE | 3-PHE | 3-PHE | 3-PHE | (X2) | Perfluoroethane-1,2-diyl group |
| (1-89) | 4-PHE | 4-PHE | 4-PHE | 4-PHE | (X2) | Perfluoroethane-1,2-diyl group |
| (1-90) | 9-PHE | 9-PHE | 9-PHE | 9-PHE | (X2) | Perfluoroethane-1,2-diyl group |

**[Table 10]**

| Compound | Ar¹ | Ar² | Ar³ | Ar⁴ | X | A |
|---|---|---|---|---|---|---|
| (1-91) | Ph | Ph | Ph | Ph | (X2) | Perfluoropropane-1,3-diyl group |
| (1-92) | 1-NAP | 1-NAP | 1-NAP | 1-NAP | (X2) | Perfluoropropane-1,3-diyl group |
| (1-93) | 2-NAP | 2-NAP | 2-NAP | 2-NAP | (X2) | Perfluoropropane-1,3-diyl group |
| (1-94) | 1-ANT | 1-ANT | 1-ANT | 1-ANT | (X2) | Perfluoropropane-1,3-diyl group |
| (1-95) | 2-ANT | 2-ANT | 2-ANT | 2-ANT | (X2) | Perfluoropropane-1,3-diyl group |
| (1-96) | 1-PHE | 1-PHE | 1-PHE | 1-PHE | (X2) | Perfluoropropane-1,3-diyl group |
| (1-97) | 2-PHE | 2-PHE | 2-PHE | 2-PHE | (X2) | Perfluoropropane-1,3-diyl group |
| (1-98) | 3-PHE | 3-PHE | 3-PHE | 3-PHE | (X2) | Perfluoropropane-1,3-diyl group |
| (1-99) | 4-PHE | 4-PHE | 4-PHE | 4-PHE | (X2) | Perfluoropropane-1,3-diyl group |
| (1-100) | 9-PHE | 9-PHE | 9-PHE | 9-PHE | (X2) | Perfluoropropane-1,3-diyl group |

**[Table 11]**

| Compound | Ar¹ | Ar² | Ar³ | Ar⁴ | X | A |
|---|---|---|---|---|---|---|
| (1-101) | Ph | Ph | Ph | Ph | (X2) | Perfluoropropane-2,2-diyl group |
| (1-102) | 1-NAP | 1-NAP | 1-NAP | 1-NAP | (X2) | Perfluoropropane-2,2-diyl group |
| (1-103) | 2-NAP | 2-NAP | 2-NAP | 2-NAP | (X2) | Perfluoropropane-2,2-diyl group |
| (1-104) | 1-ANT | 1-ANT | 1-ANT | 1-ANT | (X2) | Perfluoropropane-2,2-diyl group |
| (1-105) | 2-ANT | 2-ANT | 2-ANT | 2-ANT | (X2) | Perfluoropropane-2,2-diyl group |
| (1-106) | 1-PHE | 1-PHE | 1-PHE | 1-PHE | (X2) | Perfluoropropane-2,2-diyl group |
| (1-107) | 2-PHE | 2-PHE | 2-PHE | 2-PHE | (X2) | Perfluoropropane-2,2-diyl group |
| (1-108) | 3-PHE | 3-PHE | 3-PHE | 3-PHE | (X2) | Perfluoropropane-2,2-diyl group |
| (1-109) | 4-PHE | 4-PHE | 4-PHE | 4-PHE | (X2) | Perfluoropropane-2,2-diyl group |
| (1-110) | 9-PHE | 9-PHE | 9-PHE | 9-PHE | (X2) | Perfluoropropane-2,2-diyl group |

**[Table 12]**

| Compound | Ar¹ | Ar² | Ar³ | Ar⁴ | X | A |
|---|---|---|---|---|---|---|
| (1-111) | Ph | Ph | Ph | Ph | (X2) | Perfluorobutane-1,4-diyl group |
| (1-112) | 1-NAP | 1-NAP | 1-NAP | 1-NAP | (X2) | Perfluorobutane-1,4-diyl group |
| (1-113) | 2-NAP | 2-NAP | 2-NAP | 2-NAP | (X2) | Perfluorobutane-1,4-diyl group |
| (1-114) | 1-ANT | 1-ANT | 1-ANT | 1-ANT | (X2) | Perfluorobutane-1,4-diyl group |
| (1-115) | 2-ANT | 2-ANT | 2-ANT | 2-ANT | (X2) | Perfluorobutane-1,4-diyl group |
| (1-116) | 1-PHE | 1-PHE | 1-PHE | 1-PHE | (X2) | Perfluorobutane-1,4-diyl group |
| (1-117) | 2-PHE | 2-PHE | 2-PHE | 2-PHE | (X2) | Perfluorobutane-1,4-diyl group |
| (1-118) | 3-PHE | 3-PHE | 3-PHE | 3-PHE | (X2) | Perfluorobutane-1,4-diyl group |
| (1-119) | 4-PHE | 4-PHE | 4-PHE | 4-PHE | (X2) | Perfluorobutane-1,4-diyl group |
| (1-120) | 9-PHE | 9-PHE | 9-PHE | 9-PHE | (X2) | Perfluorobutane-1,4-diyl group |

**[Table 13]**

| Compound | Ar¹ | Ar² | Ar³ | Ar⁴ | X | A |
|---|---|---|---|---|---|---|
| (1-121) | Ph | Ph | Ph | Ph | (X2) | Perfluoropentane-1,5-diyl group |
| (1-122) | 1-NAP | 1-NAP | 1-NAP | 1-NAP | (X2) | Perfluoropentane-1,5-diyl group |
| (1-123) | 2-NAP | 2-NAP | 2-NAP | 2-NAP | (X2) | Perfluoropentane-1,5-diyl group |
| (1-124) | 1-ANT | 1-ANT | 1-ANT | 1-ANT | (X2) | Perfluoropentane-1,5-diyl group |
| (1-125) | 2-ANT | 2-ANT | 2-ANT | 2-ANT | (X2) | Perfluoropentane-1,5-diyl group |
| (1-126) | 1-PHE | 1-PHE | 1-PHE | 1-PHE | (X2) | Perfluoropentane-1,5-diyl group |
| (1-127) | 2-PHE | 2-PHE | 2-PHE | 2-PHE | (X2) | Perfluoropentane-1,5-diyl group |
| (1-128) | 3-PHE | 3-PHE | 3-PHE | 3-PHE | (X2) | Perfluoropentane-1,5-diyl group |
| (1-129) | 4-PHE | 4-PHE | 4-PHE | 4-PHE | (X2) | Perfluoropentane-1,5-diyl group |
| (1-130) | 9-PHE | 9-PHE | 9-PHE | 9-PHE | (X2) | Perfluoropentane-1,5-diyl group |

**[Table 14]**

| Compound | Ar¹ | Ar² | Ar³ | Ar⁴ | X | A |
|---|---|---|---|---|---|---|
| (1-131) | Ph | Ph | Ph | Ph | (X2) | Perfluorohexane-1,6-diyl group |
| (1-132) | 1-NAP | 1-NAP | 1-NAP | 1-NAP | (X2) | Perfluorohexane-1,6-diyl group |
| (1-133) | 2-NAP | 2-NAP | 2-NAP | 2-NAP | (X2) | Perfluorohexane-1,6-diyl group |
| (1-134) | 1-ANT | 1-ANT | 1-ANT | 1-ANT | (X2) | Perfluorohexane-1,6-diyl group |
| (1-135) | 2-ANT | 2-ANT | 2-ANT | 2-ANT | (X2) | Perfluorohexane-1,6-diyl group |
| (1-136) | 1-PHE | 1-PHE | 1-PHE | 1-PHE | (X2) | Perfluorohexane-1,6-diyl group |
| (1-137) | 2-PHE | 2-PHE | 2-PHE | 2-PHE | (X2) | Perfluorohexane-1,6-diyl group |
| (1-138) | 3-PHE | 3-PHE | 3-PHE | 3-PHE | (X2) | Perfluorohexane-1,6-diyl group |
| (1-139) | 4-PHE | 4-PHE | 4-PHE | 4-PHE | (X2) | Perfluorohexane-1,6-diyl group |
| (1-140) | 9-PHE | 9-PHE | 9-PHE | 9-PHE | (X2) | Perfluorohexane-1,6-diyl group |

**[Table 15]**

| Compound | Ar¹ | Ar² | Ar³ | Ar⁴ | X | A |
|---|---|---|---|---|---|---|
| (1-141) | Ph | Ph | Ph | Ph | (X3) | Perfluoromethanediyl group |
| (1-142) | 1-NAP | 1-NAP | 1-NAP | 1-NAP | (X3) | Perfluoromethanediyl group |
| (1-143) | 2-NAP | 2-NAP | 2-NAP | 2-NAP | (X3) | Perfluoromethanediyl group |
| (1-144) | 1-ANT | 1-ANT | 1-ANT | 1-ANT | (X3) | Perfluoromethanediyl group |
| (1-145) | 2-ANT | 2-ANT | 2-ANT | 2-ANT | (X3) | Perfluoromethanediyl group |
| (1-146) | 1-PHE | 1-PHE | 1-PHE | 1-PHE | (X3) | Perfluoromethanediyl group |
| (1-147) | 2-PHE | 2-PHE | 2-PHE | 2-PHE | (X3) | Perfluoromethanediyl group |
| (1-148) | 3-PHE | 3-PHE | 3-PHE | 3-PHE | (X3) | Perfluoromethanediyl group |
| (1-149) | 4-PHE | 4-PHE | 4-PHE | 4-PHE | (X3) | Perfluoromethanediyl group |
| (1-150) | 9-PHE | 9-PHE | 9-PHE | 9-PHE | (X3) | Perfluoromethanediyl group |

**[Table 16]**

| Compound | Ar¹ | Ar² | Ar³ | Ar⁴ | X | A |
|---|---|---|---|---|---|---|
| (1-151) | Ph | Ph | Ph | Ph | (X3) | Perfluoropropane-2,2-diyl group |
| (1-152) | 1-NAP | 1-NAP | 1-NAP | 1-NAP | (X3) | Perfluoropropane-2,2-diyl group |
| (1-153) | 2-NAP | 2-NAP | 2-NAP | 2-NAP | (X3) | Perfluoropropane-2,2-diyl group |
| (1-154) | 1-ANT | 1-ANT | 1-ANT | 1-ANT | (X3) | Perfluoropropane-2,2-diyl group |
| (1-155) | 2-ANT | 2-ANT | 2-ANT | 2-ANT | (X3) | Perfluoropropane-2,2-diyl group |
| (1-156) | 1-PHE | 1-PHE | 1-PHE | 1-PHE | (X3) | Perfluoropropane-2,2-diyl group |
| (1-157) | 2-PHE | 2-PHE | 2-PHE | 2-PHE | (X3) | Perfluoropropane-2,2-diyl group |
| (1-158) | 3-PHE | 3-PHE | 3-PHE | 3-PHE | (X3) | Perfluoropropane-2,2-diyl group |
| (1-159) | 4-PHE | 4-PHE | 4-PHE | 4-PHE | (X3) | Perfluoropropane-2,2-diyl group |
| (1-160) | 9-PHE | 9-PHE | 9-PHE | 9-PHE | (X3) | Perfluoropropane-2,2-diyl group |

**[Table 17]**

| Compound | Ar¹ | Ar² | Ar³ | Ar⁴ | X | A |
|---|---|---|---|---|---|---|
| (1-161) | Ph | Ph | Ph | Ph | (X3) | Perfluoroethane-1,2-diyl group |
| (1-162) | 1-NAP | 1-NAP | 1-NAP | 1-NAP | (X3) | Perfluoroethane-1,2-diyl group |
| (1-163) | 2-NAP | 2-NAP | 2-NAP | 2-NAP | (X3) | Perfluoroethane-1,2-diyl group |
| (1-164) | 1-ANT | 1-ANT | 1-ANT | 1-ANT | (X3) | Perfluoroethane-1,2-diyl group |
| (1-165) | 2-ANT | 2-ANT | 2-ANT | 2-ANT | (X3) | Perfluoroethane-1,2-diyl group |
| (1-166) | 1-PHE | 1-PHE | 1-PHE | 1-PHE | (X3) | Perfluoroethane-1,2-diyl group |
| (1-167) | 2-PHE | 2-PHE | 2-PHE | 2-PHE | (X3) | Perfluoroethane-1,2-diyl group |
| (1-168) | 3-PHE | 3-PHE | 3-PHE | 3-PHE | (X3) | Perfluoroethane-1,2-diyl group |
| (1-169) | 4-PHE | 4-PHE | 4-PHE | 4-PHE | (X3) | Perfluoroethane-1,2-diyl group |
| (1-170) | 9-PHE | 9-PHE | 9-PHE | 9-PHE | (X3) | Perfluoroethane-1,2-diyl group |

**[Table 18]**

| Compound | Ar¹ | Ar² | Ar³ | Ar⁴ | X | A |
|---|---|---|---|---|---|---|
| (1-171) | Ph | Ph | Ph | Ph | (X3) | Perfluoropropane-2,2-diyl group |
| (1-172) | 1-NAP | 1-NAP | 1-NAP | 1-NAP | (X3) | Perfluoropropane-2,2-diyl group |
| (1-173) | 2-NAP | 2-NAP | 2-NAP | 2-NAP | (X3) | Perfluoropropane-2,2-diyl group |
| (1-174) | 1-ANT | 1-ANT | 1-ANT | 1-ANT | (X3) | Perfluoropropane-2,2-diyl group |
| (1-175) | 2-ANT | 2-ANT | 2-ANT | 2-ANT | (X3) | Perfluoropropane-2,2-diyl group |
| (1-176) | 1-PHE | 1-PHE | 1-PHE | 1-PHE | (X3) | Perfluoropropane-2,2-diyl group |
| (1-177) | 2-PHE | 2-PHE | 2-PHE | 2-PHE | (X3) | Perfluoropropane-2,2-diyl group |
| (1-178) | 3-PHE | 3-PHE | 3-PHE | 3-PHE | (X3) | Perfluoropropane-2,2-diyl group |
| (1-179) | 4-PHE | 4-PHE | 4-PHE | 4-PHE | (X3) | Perfluoropropane-2,2-diyl group |
| (1-180) | 9-PHE | 9-PHE | 9-PHE | 9-PHE | (X3) | Perfluoropropane-2,2-diyl group |

### [Method for synthesis of triarylamine derivative]

The triarylamine derivative according to the present invention can be synthesized by reacting, in the presence of a catalyst, an amine compound represented by formula (3) with halogenated compounds represented by formulae (4) to (7). This reaction takes place in conformity with Scheme A shown below. 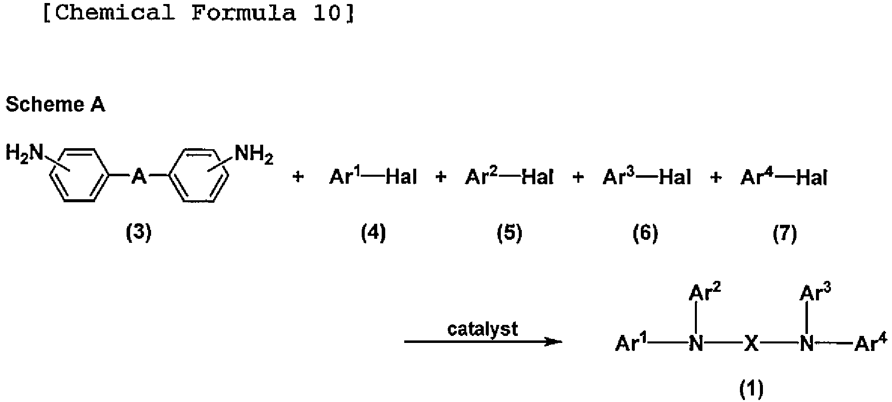 (In the formula, Ar¹ to Ar⁴, A, and X are defined as above, and Hal represents a halogen atom or pseudohalogen group.)

The triarylamine derivative according to the present invention can also be synthesized by reacting, in the presence of a catalyst, a halogenated compound represented by formulae (3') with amine compounds represented by formulae (4') and (5'). This reaction takes place in conformity with Scheme B shown below. 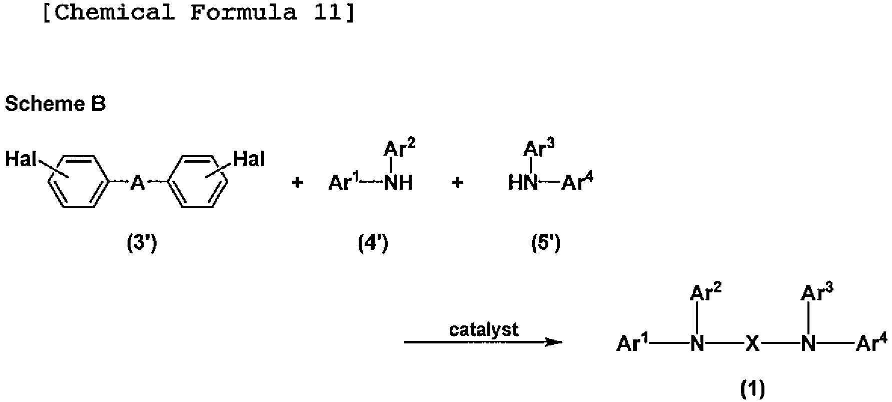 (In the formula, Ar¹ to Ar⁴, A, X, and Hal are defined as above.)

The halogen atom includes fluorine atom, chlorine atom, bromine atom, or iodine atom. The pseudohalogen group includes fluoroalkylsulfonyloxy groups such as methanesulfonyloxy group, trifluoromethanesulfonyloxy group, and nonafluorobutanesulfonyloxy group, or aromatic sulfonyloxy group such as benzenesuflonyloxy group and toluenesulfonyloxy group.

The amine compound and halogenated compound should be charged such that the total amount of "Hal" groups in all the halogenated compounds is at least one equivalent for the total amount of NH groups in all the amine compounds. The amount of "Hal" groups should preferably range from 1 to 1.2 equivalents.

Examples of the catalyst used for the above-mentioned reaction include copper catalyst such as copper chloride, copper bromide, and copper iodide, and palladium catalyst such as tetrakis(triphenylphosphine)palladium (Pd(PPh₃)₄), bis(triphenylphosphine)dichloropalladium (Pd(PPh₃)₂Cl₂), bis(benzylideneacetone)palladium (Pd(dba)₂), tris(benzylideneacetone)palladium (Pd₂(dba)₃), and bis(tri-t-butylphosphine)palladium (Pd(P-t-Bu₃)₂). These catalysts may be used alone or in combination with at least two of them. Also, these catalysts may be used in combination with any known adequate ligand.

The catalyst may be used in an amount of approximately 0.2 mol, preferably 0.15 mol, for 1 mol of the halogenated compound.

The amount of the ligand, if used, should be 0.1 to 5 equivalents, preferably 1 to 2 equivalents, for the amount of the metal complex.

The above-mentioned reactions may be performed in a solvent. In the case where the solvent is used, its type is not restricted so long as it is not detrimental to the reactions. Typical examples of the solvent include aliphatic hydrocarbons (such as pentane, n-hexane, n-octane, n-decane, and decalin), halogenated aliphatic hydrocarbons (such as chloroform, dichloromethane, dichloroethane, and carbon tetrachloride), aromatic hydrocarbons (such as benzene, nitrobenzene, toluene, o-xylene, m-xylene, p-xylene, and mesitylene), halogenated aromatic hydrocarbons (such as chlorobenzene, bromobenzene, o-dichlorobenzene, m-dichlorobenzene, and p-dichlorobenzene), ethers (such as diethyl ether, diisopropyl ether, t-butyl methyl ether, tetrahydrofuran, dioxane, 1,2-dimethoxyethane, and 1,2-diethoxyethane), ketones (such as acetone, methyl ethyl ketone, methyl isobutyl ketone, di-n-butyl ketone, and cyclohexane), amides (such as N,N-dimethylformamide and N,N-dimethyl-acetamide), lactams and lactones (such as N-methylpyrrolidone and γ-butyrolactone), ureas (such as N,N-dimethylimidazolizinone and tetramethylurea), sulfoxides (such as dimethylsulfoxide and sulfolane), and nitriles (such as acetonitrile, propionitrile, and butyronitrile). These solvents may be used alone or in combination with at least two of them.

The reaction may be performed at an appropriate temperature ranging from the solvent's melting point to boiling point, particularly, preferably approximately 0° C to 200° C, more preferably 20° C to 150° C.

After its completion, the reaction may be followed by post-treatment in the usual way to give the triarylamine derivative as desired.

### [Charge transporting varnish]

The charge transporting varnish according to the present invention contains the triarylamine derivative mentioned above, the charge transporting substance, and the organic solvent.

### [Charge transporting substance]

The charge transporting substance may be selected from those which have been conventionally used in the field of organic EL elements. Typical examples of the charge transporting substance include the following various charge transporting compound: arylamine derivatives (or aniline derivatives) such as oligoaniline derivative, N,N'-diarylbenzidine derivative, and N,N,N',N'-tetraarylbenzidine derivative, and thiophene derivatives such as oligothiophene derivative, thienothiophene derivative, and thienobenzothiophene derivative. Preferable among these examples are aniline derivatives and thiophene derivatives, with the former being more preferable.

The charge transporting compound according to the present invention should preferably have a molecular weight of approximately 200 to 9,500, so that it gives a uniform varnish for an almost perfectly flat thin film. The molecular weight should be at least 300, preferably at least 400, so that the resulting varnish gives rise to a thin film superior in charge transportability. In addition, the molecular weight should be up to 8,000, preferably 7,000, more preferably 6,000, much more preferably 5,000, so that the resulting varnish constantly gives rise to an almost perfectly flat thin film. Incidentally, the charge transporting compound should preferably be one which has no molecular weight distribution (or which has a dispersion degree of 1 or which has a single molecular weight) so that it will not separate when the charge transporting varnish is made into a thin film.

Examples of the aniline derivative include oligoaniline derivative disclosed in JP-A 2002-151272, oligoaniline compound disclosed in WO 2004/105446, oligoaniline compound disclosed in WO 2008-032617, oligoaniline compound disclosed in WO 2008/032616, and aryldiamine compound disclosed in WO 2013/042623.

In addition, the aniline derivatives represented by formula (8) below are also useful:

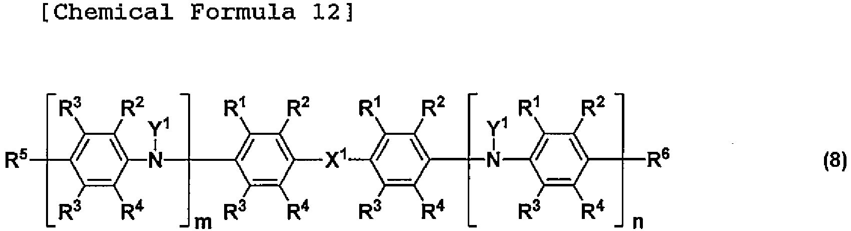

In formula (8), X¹ represents -NY¹- , -O-, -S-, -(CR⁷R⁸)_{L}-, or a single bond. However, when letter m or letter n is an integer of 0, X¹ represents -NY¹-.

Y¹ each independently represents a hydrogen atom, an alkyl group having 1 to 20 carbon atoms, alkenyl group having 2 to 20 carbon atoms, or alkynyl group having 2 to 20 carbon atoms which may be substituted with Z¹; or an aryl group having 6 to 20 carbon atoms or heteroaryl group having 2 to 20 carbon atoms which may be substituted with Z².

The alkyl group having 1 to 20 carbon atoms may be any of linear, branched, and cyclic ones. Their examples include linear or branched alkyl groups having 1 to 20 carbon atoms such as methyl group, ethyl group, n-propyl group, isopropyl group, n-butyl group, isobutyl group, s-butyl group, t-butyl group, n-pentyl group, n-hexyl group, n-heptyl group, n-octyl group, n-nonyl group, and n-decyl group, and cyclic alkyl groups having 3 to 20 carbon atoms such as cyclopropyl group, cyclobutyl group, cyclopentyl group, cyclohexyl group, cycloheptyl group, cyclooctyl group, cyclononyl group, cyclodecyl group, bicyclobutyl group, bicyclopentyl group, bicyclohexyl group, bicycloheptyl group, bicyclooctyl group, bicyclononyl group, and bicycle-decyl group.

The alkenyl group having 2 to 20 carbon atoms may be any of linear, branched, and cyclic ones. Their examples include ethenyl group, n-1-propenyl group, n-2-propenyl group, 1-methylethenyl group, n-1-butenyl group, n-2-butenyl group, n-3-butenyl group, 2-methyl-1-propenyl group, 2-methyl-2-propenyl group, 1-ethylethenyl group, 1-methyl-1-propenyl group, 1-methyl-2-propenyl group, n-1-pentenyl group, n-1-decenyl group, and n-1-eicocenyl group.

The alkynyl group having 2 to 20 carbon atoms may be any of linear, branched, and cyclic ones. Their examples include ethynyl group, n-1-propynyl group, n-2-propynyl group, n-1-butynyl group, n-2-butynyl group, n-3-butynyl group, 1-methyl-2-propynyl group, n-1-pentynyl group, n-2-pentynyl group, n-3-pentynyl group, n-4-pentynyl group, 1-methyl-n-butynyl group, 2-methyl-n-butynyl group, 3-methyl-n-butynyl group, 1,1-di-methyl-n-propynyl group, n-1-hexynyl group, n-1-decynyl group, n-1-pentadecynyl group, and n-1-eicocynyl group.

Examples of the aryl group having 6 to 20 carbon atoms include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, and 9-phenanthryl group.

Examples of the heteroaryl group having 2 to 20 carbon atoms include 2-thienyl group, 3-thienyl group, 2-furanyl group, 3-furanyl group, 2-oxazolyl group, 4-oxazolyl group, 5-oxazolyl group, 3-isooxazolyl group, 4-isooxazolyl group, 5-isooxazolyl group, 2-thiazolyl group, 4-thiazolyl group, 5-thiazolyl group, 3-isothiazolyl group, 4-isothiazolyl group, 5-isothiazolyl group, 2-imidazolyl group, 4-imidazolyl group, 2-pyridyl group, 3-pyridyl group, and 4-pyridyl group.

R⁷ and R⁸ each independently represent a hydrogen atom, halogen atom, nitro group, cyano group, amino group, aldehyde group, hydroxyl group, thiol group, sulfonic group, carboxyl group; any of alkyl group having 1 to 20 carbon atoms, alkenyl group having 2 to 20 carbon atoms, and alkynyl group having 2 to 20 carbon atoms which may have an optional substituent Z¹; any of aryl group having 6 to 20 carbon atoms and heteroaryl group having 2 to 20 carbon atoms which may have an optional substituent Z²; any of -NHY², -NY³Y⁴, -C(O)Y⁵, -OY⁶, -SY⁷, -SO₃Y⁸, -C(O)OY⁹, -OC(O)Y¹⁰, -C(O)NHY¹¹, and -C(O)NY¹²Y¹³.

Y² to Y¹³ each independently represent any of alkyl group having 1 to 20 carbon atoms, alkenyl group having 2 to 20 carbon atoms, and alkynyl group having 2 to 20 carbon atoms which may have an optional substituent Z¹; or any of aryl group having 6 to 20 carbon atoms and heteroaryl group having 2 to 20 carbon atoms which may have an optional substituent Z².

Z¹ represents a halogen atom, nitro group, cyano group, amino group, aldehyde group, hydroxyl group, thiol group, sulfonic group, carboxyl group, or any of aryl group having 6 to 20 carbon atoms and heteroaryl group having 2 to 20 carbon atoms which may have an optional substituent Z³.

Z² represents a halogen atom, nitro group, cyano group, amino group, aldehyde group, hydroxyl group, thiol group, sulfonic group, carboxyl group, or any of alkyl group having 1 to 20 carbon atoms, alkenyl group having 2 to 20 carbon atoms, and alkynyl group having 2 to 20 carbon atoms which may have an optional substituent Z³.

Z³ represents a halogen atom, nitro group, cyano group, amino group, aldehyde group, hydroxyl group, thiol group, sulfonic group, or carboxyl group.

The halogen atom includes fluorine atom, chlorine atom, bromine atom, or iodine atom.

The alkyl group, alkenyl group, alkynyl group, aryl group, and heteroaryl group represented by R⁷ and R⁸ and Y² to Y¹³ are the same ones as mentioned above.

Preferable among those represented by R⁷ and R⁸ are a hydrogen atom and alkyl groups having 1 to 20 carbon atoms which may have an optional substituent Z¹. More preferable among them are a hydrogen atom or a methyl group which may have an optional substituent Z¹. The most desirable is one in which both R⁷ and R⁸ are hydrogen atoms.

L represents the number of groups represented by -(CR⁷R⁸)- and it should be an integer of 1 to 20, preferably 1 to 10, more preferably 1 to 5, much more preferably 1 or 2, and most desirably 1. If L is at least 2, a plurality of groups R⁷ may be mutually identical or different, and a plurality of group R⁸ may be mutually identical or different.

Particularly preferable examples of X¹ include -NY¹- and single bond. Preferable among those represented by Y¹ are a hydrogen atom and alkyl groups having 1 to 20 carbon atoms which may have an optional substituent Z¹. More preferable among them are a hydrogen atom or a methyl group which may have an optional substituent Z¹. The most desirable is one in which Y¹ is a hydrogen atom.

R¹ to R⁶ each independently represent a hydrogen atom, halogen atom, nitro group, cyano group, amino group, aldehyde group, hydroxyl group, thiol group, sulfonic group, carboxyl group; any of alkyl group having 1 to 20 carbon atoms, alkenyl group having 2 to 20 carbon atoms, and alkynyl group having 2 to 20 carbon atoms which may have an optional substituent Z¹; any of aryl group having 6 to 20 carbon atoms and heteroaryl group having 2 to 20 carbon atoms which may have an optional substituent Z²; any of -NHY², -NY³Y⁴, -C(O)Y⁵, -OY⁶, -SY⁷, -SO₃Y⁸, -C(O)OY⁹, -OC(O)Y¹⁰, -C(O)NHY¹¹, and -C(O)NY¹²Y¹³ (wherein Y² to Y¹³ are defined as above). The same examples as mentioned above are listed for the halogen atom, alkyl group, alkenyl group, alkynyl group, aryl group, and heteroaryl group.

In formula (8), particularly preferable examples of R¹ to R⁴ are a hydrogen atom, halogen atom, alkyl groups having 1 to 10 carbon atoms which may have an optional substituent Z¹, and aryl groups having 6 to 14 carbon atoms which may have an optional substituent Z². More preferable among them are a hydrogen atom, fluorine atom, and alkyl groups having 1 to 10 carbon atoms which may optionally be substituted with a fluorine atom. The most desirable is one in which all of them are hydrogen atoms.

Among preferable examples of R⁵ and R⁶ are a hydrogen atom, halogen atom, alkyl group having 1 to 10 carbon atoms which may have an optional substituent Z¹, aryl group having 6 to 14 carbon atoms which may have an optional substituent Z², and diphenyl amino group which may have an optional substituent Z² (or -NY³Y⁴ group in which Y³ and Y⁴ are phenyl groups optionally substituted by Z²). More preferable among them are a hydrogen atom, fluorine atom, and diphenylamino group which may optionally be substituted with a fluorine atom. The most desirable is one in which all of them are hydrogen atoms or diphenylamino groups simultaneously.

Among preferable examples are those in which each of R¹ to R⁴ is a hydrogen atom, fluorine atom, alkyl group having 1 to 10 carbon atoms optionally substituted with a fluorine atom, each of R⁵ and R⁶ is a hydrogen atom, fluorine atom, or diphenylamino group optionally substituted with a fluorine atom, X¹ is -NY¹- or a single bond, and Y¹ is a hydrogen atom or methyl group. Among more preferable examples are those in which each of R¹ to R⁴ is a hydrogen atom, R⁵ and R⁶ are hydrogen atoms or diphenylamino groups simultaneously, and X¹ is -NH- or a single bond.

In formula (8), letters m and n each independently represent an integer of at least 0, in the range of 1 ≤ m+n ≤ 20. The range should preferably be 2 ≤ m+n ≤ 8, more preferably 2 ≤ m+n ≤ 6, and most desirably 2 ≤ m+n ≤ 4, from the standpoint of balance between the charge transportability of the resulting thin film and the solubility of the aniline derivative.

Particularly desirable examples of Y¹ to Y¹³ and R¹ to R⁸ are those in which Z¹ is a halogen atom or an aryl group having 6 to 20 carbon atoms which may have an optional substituent Z³. Much more preferable examples are those in which Z¹ is a halogen atom or a phenyl group which may have an optional substituent Z³. The most desirable examples are those in which there exist no substituents.

Examples of Z² include a halogen atom or an alkyl group having 1 to 20 carbon atoms which may have an optional substituent Z³. More preferable examples of Z² include a halogen atom or an alkyl group having 1 to 4 carbon atoms which may have an optional substituent Z³. Most preferable examples of Z² are those in which there exist no substituents.

Examples of Z³ are halogen atoms, preferably fluorine. Most desirable examples are those in which there exist no substituents.

In Y¹ to Y¹³ and R¹ to R⁸, the alkyl group, alkenyl group, and alkynyl group should have a carbon number up to 10, preferably up to 6, and more preferably up to 4. In addition, the aryl group and heteroaryl group should have a carbon number up to 14, preferably up to 10, and more preferably up to 6.

The aniline derivative represented by formula (8) above should usually have a molecular weight of 300 to 5,000. For better solubility, the molecular weight should preferably be up to 4,000, more preferably up to 3,000, and most desirably up to 2,000.

Incidentally, the aniline derivative mentioned above is not specifically restricted in its synthesis method. Some examples of the synthesis method are found in the following documents: Bulletin of Chemical Society of Japan, 67, pp. 1749-1752 (1994); Synthetic Materials, 84, pp. 119-120 (1997); Thin Solid Films, 520(24), pp. 7157-7163 (2012); WO 2008/032617; WO 2008/032616; and WO 2008/129947.

Examples of the aniline derivative represented by formula (8) typically include, but are not limited to, those which are represented by the following formulas, in which DPA represents a diphenylamino group, Ph represents a phenyl group, TPA represents a p-(diphenylamino)phenyl group, and Np represents a 1-naphthyl group.

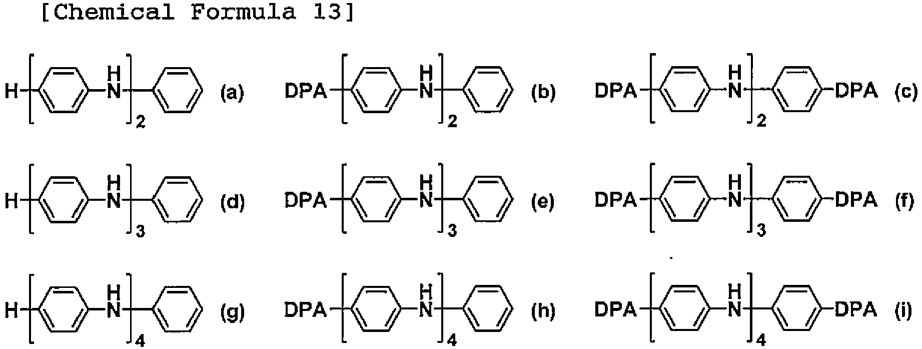

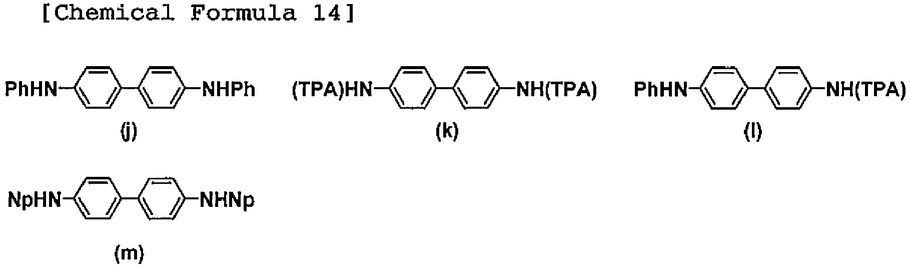

The varnish according to the present invention should include the triarylamine derivative of the present invention in a specific amount which varies depending on the type and quantity of the charge transporting substance and dopant and the charge transportability required, therefore it cannot be flatly prescribed. The amount in terms of the ratio to the total amount of the charge transporting substance and dopant is usually approximately 0.1 to 50 wt%, preferably approximately 0.5 to 40 wt%, more preferably approximately 0.8 to 30 wt%, and most desirably approximately 1 to 20 wt%.

### [Organic solvent]

The charge transporting varnish may be prepared by using an organic solvent capable of readily dissolving the charge transporting substance and dopant.

Examples of the organic solvent unrestrictedly include cyclohexane, N,N-dimethylformamide, N,N-dimethylacetamide, N-methylpyrrolidone, and 1,3-dimethyl-2-imidazolidinone. They may be used alone or in combination of at least two of them. The amount of the organic solvent should be 5 to 100 wt% in all the solvents used for the varnish.

Incidentally, the above solvent should be used to achieve complete dissolution or uniform dispersion therein for the charge transporting substance and dopant. Complete dissolution is preferable.

Moreover, the varnish according to the present invention may additionally include at least one kind of high-viscosity organic solvent which has a viscosity of 10 to 200 mPa·s, particularly 35 to 150 mPa·s, at 25°C, and a boiling point of 50 to 300°C, particularly 150 to 250°C, at normal pressure (atmospheric pressure). This solvent makes it easy to adjust the viscosity of the varnish. The easy viscosity adjustment facilitates preparation of any varnish suitable for the coating which gives rise to highly flat thin films with good reproducibility.

Examples of the high-viscosity organic solvent unrestrictedly include cyclohexanol, ethylene glycol, ethylene glycol diglycidyl ether, 1,3-octylene glycol, diethylene glycol, dipropylene glycol, triethylene glycol, tripropylene glycol, 1,3-butanediol, 2,3-butanediol, 1,4-butanediol, propylene glycol, and hexylene glycol.

The amount of the high-viscosity organic solvent which accounts for in the entire solvent used for the varnish according to the present invention should be 5 to 80 wt%, so long as solid precipitation does not occur.

Moreover, the varnish may include an additional solvent for improvement in substrate wettability, adjustment of solvent's surface tension, adjustment of polarity, and adjustment of boiling point. The additional solvent should be used in an amount of 1 to 90 wt%, preferably 1 to 50 wt%, of the entire solvents in the varnish.

Examples of the additional solvent unrestrictedly include propylene glycol monomethyl ether, ethylene glycol monobutyl ether, diethylene glycol diethyl ether, diethylene glycol monomethyl ether, diethylene glycol dimethyl ether, diethylene glycol monoethyl ether acetate, diethylene glycol monobutyl ether acetate, dipropylene glycol monomethyl ether, propylene glycol monomethyl ether acetate, diethylene glycol monoethyl ether, diacetone alcohol, γ-butyrolactone, ethyl lactate, and n-hexyl acetate. They may be used alone or in combination of at least two of them.

The varnish according to the present invention should have an adequate viscosity in conformity with the thickness of the thin film to be prepared and the concentration of solids therein. The viscosity usually ranges from 1 to 50 mPa·s at 25°C. The composition according to the present invention should have an adequate concentration of solids which varies depending on its viscosity, surface tension and the thickness of the thin film to be prepared. The concentration usually ranges from approximately 0.1 to 20.0 wt%, preferably 0.5 to 10.0 wt%, more preferably 1.0 to 5.0 wt%, for better spreadability. The term "solids" used therein embraces the charge transporting substance and dopant included in the composition according to the present invention.

### [Dopant]

The charge transporting varnish according to the present invention may optionally include a dopant for improvement in charge transportability depending on the application of the thin film to be formed. The dopant is not specifically restricted so long as it is soluble in at least one solvent used for the varnish. The dopant may be either inorganic one or organic one.

The dopant may be used in varied amounts depending on its type. An adequate amount in the ratio to the unit weight of the charge transporting substance is usually approximately 0.5 to 10.0, preferably 3.0 to 9.0.

Examples of the inorganic dopant include inorganic acids such as hydrogen chloride, sulfuric acid, nitric acid, and phosphoric acid; metal halides such as aluminum (III) chloride (AlCl₃), titanium (IV) tetrachloride (TiCl₄), boron tribromide (BBr₃), boron trifluoride-ether complex (BF₃·OEt₂), iron (III) chloride (FeCl₃), copper (II) chloride (CuCl₂), antimony (V) pentachloride (SbCl₅), antimony (V) pentafluoride (SbF₅), arsenic (V) pentafluoride (AsF₅), phosphorus pentafluoride (PF₅), and tris(4-bromophenyl)aluminum hexachloroantimonate (TBPAH); halogens such as Cl₂, Br₂, I₂, ICl, ICl₃, IBr, and IF₄; and heteropolyacids such as phosphomolybdic acid, phosphotungstic acid, silicomolybdic acid, silicotungstic acid, and phosphotungustomolybdic acid.

Examples of the organic dopant include benzenesulfonic acid, tosyl acid, p-styrenesulfonic acid, 2-naphthalenesulfonic acid, 4-hydroxybenzenesulfonic acid, 5-sulfosalicylic acid, p-dodecylbenzenesulfonic acid, dihexylbenzenesulfonic acid, 2,5-dihexylbenzenesulfonic acid, dibutylnaphthalenesulfonic acid, 6,7-dibutyl-2-naphthalenesulfonic acid, dodecylnaphthalenesulfonic acid, 3-dodecyl-2-naphthalenesulfonic acid, hexylnaphthalenesulfonic acid, 4-hexyl-1-naphthalenesulfonic acid, octylnaphthalenesulfonic acid, 2-octyl-1-naphthalenesulfonic acid, hexylnaphthalene sulfonic acid, 7-hexyl-1-naphthalenesulfonic acid, 6-hexyl-2-naphthalenesulfonic acid, dinonylnaphthalenesulfonic acid, 2,7-dinonyl-4-naphthalenesulfonic acid, dinonylnaphthalenedisulfonic acid and 2,7-dinonyl-4,5-naphthalenedisulfonic acid. Additional examples include 1,4-benzodioxanedisulfonic acid disclosed in WO 2005/000832, arylsulfonic acid compound disclosed in WO 2006/025342, arylsulfonic acid compound disclosed in WO 2009/096352, and arylsulfonic acid compound such as polystyrenesulfonic acid. These inorganic and organic dopants may be used alone or in combination of at least two of them.

Most effective among these dopants is heteropolyacid, which improves the hole transportability of the triarylamine derivative according to the present invention.

### [Method for preparation of charge transporting varnish]

The charge transporting varnish may be prepared in any way without specific restrictions. One method, for example, includes dissolving the triarylamine derivative and charge transporting substance according to the present invention in a high-solvency solvent, and then incorporating the resulting solution with a high-viscosity organic solvent. Another method includes mixing together a high-solvency solvent and a high-viscosity organic solvent and then dissolving in the resulting mixture the triarylamine derivative and charge transporting substance according to the present invention.

According to the present invention, the charge transporting varnish should preferably be filtered through a filter of submicron order after the triarylamine derivative and charge transporting substance according to the present invention have been dissolved in an organic solvent. This step is necessary so that the charge transporting varnish gives rise to a highly flat thin film with good reproducibility.

### [Charge transporting thin film]

The charge transporting varnish according to the present invention forms a charge transporting thin film on a substrate by applying onto a substrate, followed by baking.

Coating of the varnish may be accomplished in various ways, without specific restrictions, such as dipping, spin coating, transfer printing, roll coating, brushing, ink jet printing, spraying, and slit coating. The viscosity and the surface tension of the varnish may be preferably adjusted according to the coating.

The baking may be accomplished in various atmospheres, such as air, nitrogen or any other inert gas, and vacuum. By baking, the varnish gives rise to a uniform thin film with a high charge transportability.

The baking should be accomplished at an adequate temperature ranging from approximately 100 to 260°C in view of the use of the thin film and the charge transportability required of the thin film to be obtained. In the case where the resulting thin film is intended for use as the hole injection layer of the organic EL element, the adequate baking temperature is approximately 140 to 250° C, preferably approximately 145 to 240°C.

The baking may be accomplished in at least two stages at different temperatures. Baking in this way is effective in achieving higher uniformity and causing reactions to proceed on the substrate. Heating for baking simply needs a hot plate, oven, or the like.

The charge transporting thin film is not specifically restricted in film thickness. In the case where it is intended for use as the hole injection layer in the organic EL element, the adequate film thickness is 5 to 200 nm. The film thickness may be varied by adjusting the concentration of solids in the varnish or adjusting the amount of solution on the substrate at the time of coating.

The charge transporting thin film according to the present invention properly functions as the hole injection layer in the organic EL element. It also functions as the charge transporting functional layer, such as hole injection transporting layer.

### [Organic EL element]

The organic EL element according to the present invention includes paired electrodes and the above-mentioned charge transporting thin film according to the present invention, held between them.

The organic EL element is typically constructed of several components as exemplified (without restrictions) in the following paragraphs (a) to (f). Incidentally, the following construction may be modified, if necessary, such that an electron blocking layer is interposed between the emitting layer and the anode, and a hole blocking layer is interposed between the emitting layer and the cathode. The construction may be modified further such that the hole injection layer, the hole transporting layer, or the hole injection transporting layer functions as the electron blocking layer, and the electron injection layer, the electron transporting layer, or the electron injection transporting layer functions as the hole blocking layer.
(a) Anode/hole injection layer/hole transporting layer/emitting layer/electron transporting layer/electron injection layer/cathode
(b) Anode/hole injection layer/hole transporting layer/emitting layer/electron injection transporting layer/cathode
(c) Anode/hole injection transporting layer/emitting layer/electron transporting layer/electron injection layer/cathode
(d) Anode/hole injection transporting layer/emitting layer/electron injection transporting layer/cathode
(e) Anode/hole injection layer/hole transmitting layer/emitting layer/cathode
(f) Anode/hole injection transmitting layer/emitting layer/cathode

The terms "hole injection layer," "hole transporting layer" and "hole injection transporting layer" individually represents a layer formed between the anode and the emitting layer. This layer functions to transport holes from the anode to the emitting layer. In the case where there is only one layer of hole transporting material between the anode and the emitting layer, that layer is called "hole injection transporting layer." In the case where there exist at least two layers of hole transporting material between the anode and the emitting layer, the one closer to the anode is called "hole injection layer" and the other ones are called "hole transporting layer." The hole injection layer and hole injection transporting layer are formed from thin films superior not only in the ability to accept holes from the anode but also in the ability to transport holes into the hole transporting layer and emitting layer.

The terms "electron injection layer," "electron transporting layer" and "electron injection transporting layer" individually represents a layer formed between the cathode and the emitting layer. This layer functions to transport electrons from the cathode to the emitting layer. In the case where there is only one layer of electron transporting material between the cathode and the emitting layer, that layer is called "electron injection transporting layer." In the case where there exist at least two layers of electron transporting material between the cathode and the emitting layer, the one closer to the cathode is called "electron injection layer" and the other ones are called "electron transporting layer."

The term "emitting layer" represents an organic layer having the emitting function. It includes a host material and a dopant material in the case where it relies on the doping system. In this case, the host material mainly promotes the recombination of electrons and holes and confines excitons in the emitting layer, and the dopant material causes the excitons resulting from recombination to efficiently emit light. In the case of phosphorescent element, the host material confines the excitons generated by the dopant, in the emitting layer.

The charge transporting thin film according to the present invention appropriately functions as the hole injection layer, the hole transporting layer, and the hole injection transporting layer in the organic EL element. It functions more appropriately as the hole injection layer.

In the case where the charge transporting varnish according to the present invention is used to produce the organic EL element, various materials and methods are employed without restrictions as shown in the following.

The electrode substrate to be used should be made ready by previous cleaning with detergent, alcohol, pure water, etc. For example, the anode substrate should undergo surface treatment such as ultraviolet (UV)-ozone treatment and oxygen-plasma treatment immediately before its use. However, the anode composed mainly of organic materials does not need surface treatment.

In the case where the charge transporting varnish according to the present invention is made into a thin film as the hole injection layer, the organic EL element according to the present invention is produced by the method which is illustrated below as an example.

The process for production starts with coating the anode substrate with the charge transporting varnish according to the present invention. After baking, there is obtained the hole injection layer formed on the electrode. Thus, the hole injection layer is coated sequentially with the hole transporting layer, emitting layer, electron transporting layer, electron injection layer, and cathode. The hole transporting layer, emitting layer, electron transporting layer, and electron injection layer should be formed by either vapor deposition or coating (wet process) in conformity with the properties of the material use.

The anode may be a transparent electrode such as indium tin oxide (ITO) or indium zinc oxide (IZO) or an opaque electrode such as aluminum or any other metal or alloy thereof. The anode should preferably undergo planarization. It is also possible to use a polythiophene derivative or polyaniline derivative, which have high charge transportability.

Incidentally, the metal anode may be formed from any one of the following metals and others: scandium, titanium, vanadium, chromium, manganese, iron, cobalt, nickel, copper, zinc, gallium, yttrium, zirconium, niobium, molybdenum, ruthenium, rhodium, palladium, cadmium, indium, scandium, lanthanum, cerium, praseodymium, neodymium, promethium, samarium, europium, gadolinium, terbium, dysprosium, holmium, erbium, thulium, ytterbium, hafnium, thallium, tungsten, rhenium, osmium, iridium, platinum, gold, titanium, lead, and bismuth, and alloys thereof.

The hole transporting layer is formed from any low-molecular weight materials capable of hole transportation, such as triarylamines whose examples are listed below and oligothiophenes whose example is 5,5"-bis-{4-[bis(4-methylphenyl)amino]phenyl}-2,2':5`,2"-terthiophene (BMA-3T). Examples of triarylamines include (triphenylamine)dimer derivative, [(triphenylamine)dimer]spirodimer, N,N'-bis(naphthalen-1-yl)-N,N'-bis(phenyl)-benzidine (α-NPD), N,N'-bis(naphthalen-2-yl)-N,N'-bis(phenyl)-benzidine, N,N'-bis(3-methylphenyl)-N,N'-bis(phenyl)-benzidine, N,N'-bis(3-methylphenyl)-N,N'-bis(phenyl)-9,9-spirobifluorene, N,N'-bis(naphthalen-1-yl)-N,N'-bis(phenyl)-9,9-spirobifluorene, N,N'-bis(3-methylphenyl)-N,N'-bis(phenyl)-9,9-dimethylfluorene, N,N'-bis(naphthalen-1-yl)-N,N'-bis(phenyl)-9,9-dimethylfluorene, N,N'-bis(3-methylphenyl)-N,N'-bis(phenyl)-9,9-diphenylfluorene, N,N'-bis(naphthalen-l-yl)-N,N'-bis(phenyl)-9,9-diphenylfluorene, N,N'-bis(naphthalen-1-yl)-N,N'-bis(phenyl)-2,2'-dimethylbenzidine, 2,2',7,7'-tetrakis(N,N'-diphenylamino)-9,9-spirobifluorene, 9,9-bis[4-(N,N-bis-biphenyl-4-yl-amino)phenyl]-9H-fluorene, 9,9-bis[4-(N,N-bis-naphthalen-2-yl-amino)phenyl]-9H-fluorene, 9,9-bis[4-(N-naphthalen-1-yl-N-phenylamino)-phenyl]-9H-fluorene, 2,2',7.7'-tetrakis[N-naphthalenyl(phenyl)-amino]-9,9-spirofluorene, N,N'-bis(phenanthren-9-yl)-N,N'-bis(phenyl)-benzidine, 2,2'-bis[N,N-bis(biphenyl-4-yl)amino]-9,9-spirobifluorene, 2,2'-bis(N,N-diphenylamino)-9,9-spirobifluorene, di-[4-(N,N-di(p-tolyl)amino)-phenyl]cyclohexane, 2,2',7,7'-tetra(N,N-di(p-tolyl)amino)-9,9-spirobifluorene, N,N,N',N'-tetra-naphthalen-2-yl-benzidine, N,N,N',N'-tetra-(3-methylphenyl)-3,3-dimehtylbenzidine, N,N'-di(naphthalenyl)-N,N'-di(naphthalen-2-yl)-benzidine, N,N,N',N'-tetra(naphthalenyl)-benzidine, N,N'-di(naphthalen-2-yl)-N,N'-diphenylbenzidine-1,4-diamine, N¹,N⁴-diphenyl-N¹,N⁴-di(m-tolyl)benzene-1,4-diamine, N²,N²,N⁶,N⁶-tetraphenylnaphthalene-2,6-diamine, tris(4-(quinolin-8-yl)phenyl)amine, 2,2'-bis(3-(N,N-di(p-tolyl)amino)phenyl)biphenyl, 4,4',4"-tris[3-methylphenyl(phenyl)amino]triphenylamine (m-MTDATA), and 4,4',4"-tris[1-naphthyl(phenyl)amino]triphenylamine (1-TNATA).

Examples of materials for forming the emitting layer include tris(8-quinolinolato)aluminum(III) (Alq₃), bis(8-quinolinolato)zinc(II) (Znq₂), bis(2-methyl-8-quinolinolato)-4-(p-phenylphenolato)-aluminum(III) (BAlq), 4,4'-bis(2,2-diphenylvinyl)biphenyl, 9,10-di(naphthalen-2-yl)anthracene, 2-t-butyl-9,10-di(naphthalen-2-yl)anthracene, 2,7-bis[9,9-di(4-methylphenyl)-fluoren-2-yl]-9,9-di(4-methylphenyl)fluorene, 2-methyl-9,10-bis(naphthalen-2-yl)anthracene, 2-(9,9-spirobifluoren-2-yl)-9,9-spirobifluorene, 2,7-bis(9,9-spirobifluoren-2-yl)-9,9-spirobifluorene, 2-[9,9-di(4-methylphenyl)-fluoren-2-yl]-9,9-di(4-methylphneyl)fluorene, 2,2'-dipyrenyl-9,9-spirobifluorene, 1,3,5-tris(pyren-1-yl)benzene, 9,9-bis[4-(pyrenyl)phenyl]-9H-fluorene, 2,2'-bi(9,10-diphenylanthracene), 2,7-dipyrenyl-9,9-spirobifluorene, 1,4-di(pyren-1-yl)benzene, 1,3-di(pyren-1-yl)benzene, 6,13-di(biphenyl-4-yl)pentacene, 3,9-di(naphthalen-2-yl)perylene, 3,10-di(naphthalen-2-yl)perylene, tris[4-(pyrenyl)-phenyl]amine, 10,10'-di(biphenyl-4-yl)-9,9'"bianthracene, N,N'-di(naphthalen-1-yl)-N,N'-diphenyl-[1,1':4',1":4",1"'-quaterphenyl]-4,4"'-diamine, 4,4'-di[10-(naphthalen-1-yl)anthracen-9-yl]biphenyl, dibenzo{[f,f']-4,4',7,7'-tetraphenyl}diindeno[1,2,3-cd:1',2',3'-lm]perylene, 1-(7-(9,9'-bianthracen-10-yl)-9,9-dimethyl-9H-fluoren-2-yl)pyrene, 1-(7-(9,9'-biantharcen-10-yl)-9,9-dihexyl-9H-fluoren-2-yl)pyrene, 1,3-bis(carbazol-9-yl)benzene, 1,3,5-tris(carbazol-9-yl)benzene, 4,4',4"-tris(carbazol-9-yl)triphenylamine, 4,4'-bis(carbazol-9-yl)biphenyl (CBP), 4,4'-bis(carbazol-9-yl)-2,2'-dimethylbiphenyl, 2,7-bis(carbazol-9-yl)-9,9-dimethylfluorene, 2,2',7,7'-tetrakis(carbazol-9-yl)-9,9-spirobifluorene, 2,7-bis(carbazol-9-yl)-9,9-di(p-tolyl)fluorene, 9,9-bis[4-(carbazol-9-yl)-phenyl]fluorene, 2,7-bis(carbazol-9-yl)-9,9-spirobifluorene, 1,4-bis(triphenylsilyl)benzene, 1,3-bis(triphenylsilyl)benzene, bis(4-N,N-diethylamino-2-methylphenyl)-4-methylphenylmethane, 2,7-bis(carbazol-9-yl)-9,9-dioctylfluorene, 4,4"-di(triphenylsilyl)-p-terphenyl, 4,4'-di(triphenylsilyl)biphenyl, 9-(4-t-butylphenyl)-3,6-bis(triphenylsilyl)-9H-carbazol, 9-(4-t-butylphenyl)-3,6-ditrityl-9H-carbzole, 9-(4-t-butylphenyl)-3,6-bis(9-(4-methoxyphenyl)-9H-fluoren-9-yl)-9H-carbazole, 2,6-bis(3-(9H-cabazol-9-yl)phenyl)pyridine, triphenyl(4-(9-phenyl-9H-fluoren-9-yl)phenyl)silane, 9,9-dimethyl-N,N-diphenyl-7-(4-(1-phenyl-1H-benzo[d]imizazol-2-yl)phenyl-9H-fluorene-2-amine, 3,5-bis(3-(9H-carbazol-9-yl)phenyl)pyridine, 9,9-spirofluoren-2-yl-diphenyl-phosphine oxide, 9,9'-(5-(triphenylsilyl)-1,3-phenylene)bis(9H-carbzol), 3-(2,7-bis(diphenylphosphoryl)-9-phenyl-9H-fluoren-9-yl)-9-phenyl-9H-carbzole, 4,4,8,8,12,12-hexa(p-tolyl)-4H-8H-12H-12C-azadibenzo-[cd,mn]pyrene, 4,7-di(9H-carbzol-9-yl)-1,10-phenanthroline, 2,2'-bis(4-carbazol-9-yl)phenyl)biphenyl, 2,8-bis(diphenylphosphoryl)dibenzo[b,d]thlophene, bis(2-methylphenyl)diphenylsilane, bis[3,5-di(9H-carbazol-9-yl)phenyl]diphenylsilane, 3,6-bis(carbazol-9-yl)-9-(2-ethyl-hexyl)-9H-carbazole, 3-(diphenylphosphoryl)-9-(4-(diphenylphosphoryl)phenyl)-9H-carbazole, and 3,6-bis[(3,5-diphenyl)phenyl]-9-phenylcarbazole. The above materials may be used in combination with an emitting dopant to form the emitting layer by codeposition.

Examples of the emitting dopant include 3-(2-benzothiazolyl)-7-(diethylamino)coumarin, 2,3,6,7-tetrahydro-1,1,7,7-tetramethyl-1H,5H,11H-10-(2-benzothiazolyl)quinolizino[9,9a,1gh]coumarine, quinacridone, N,N'-dimethylquinacridone, tris(2-phenylpyridine)iridium(III) (Ir(ppy)₃), bis(2-phenylpyridine)(acetylacetonate)iridium(III) (Ir(ppy)₂(acac)), tris[2-(p-tolyl)pyridine]iridium(III) (Ir(mppy)₃), 9,10-bis[N,N-di(p-tolyl)amino]anthracene, 9,10-bis[phenyl(m-tolyl)amino]anthracene, bis[2-(2-hydroxyphenyl)benzothiazolato]zinc(II), N¹⁰,N¹⁰,N¹⁰,N¹⁰-tetra(p-tolyl)-9,9'-bianthracene-10,10'-diamine, N¹⁰,N¹⁰,N¹⁰,N¹⁰-tetraphenyl-9,9'-bianthracene-10,10'-diamine, N¹⁰,N¹⁰-diphenyl-N¹⁰,N¹⁰-dinaphthalenyl-9,9'-bianthracene-10,10'-diamine, 4,4'-bis(9-ethyl-3-carbazovinylene)-1,1'-biphenyl, perylene, 2,5,8,11-tetra-t-butylperylene, 1,4-bis[2-(3-N-ethylcarbazolyl)vinyl]benzene, 4,4'-bis[4-(di-p-tolylamino)styryl]biphenyl, 4-(di-p-tolylamino)-4'-[(di-p-tolylamino)styryl]stilbene, bis[3,5-difluoro-2-(2-pyridyl)phenyl-(2-carboxypyridyl)]-iridium(III), 4,4'-bis[4-(diphenylamino)styryl]biphenyl, bis(2,4-difluorophenylpyridinato)tetrakis(1-pyrazolyl)borate iridium(III), N,N'-bis(naphthalen-2-yl)-N,N'-bis(phenyl)-tris(9,9-dimethylfluorenylene), 2,7-bis{2-[phenyl(m-tolyl)amino]-9,9-dimethyl-fluoren-7-yl}-9,9-dimethyl-fluorene, N-(4-((E)-2-(6((E)-4-(diphenylamino)styryl)naphthalen-2-yl)vinyl)phenyl)-N-phenylbenzeneamine, fac-iridium(III)tris(1-phenyl-3-methylbenzimidazoline-2-indene-C,C²), mer-iridium(III)tris(1-phenyl-3-methylbenzimidazoline-2-indene-C,C²), 2,7-bis[4-(diphenylamino)styryl]-9,9-spirobifluorene, 6-methyl-2-(4-(9-(4-(6-methylbenzo[d]thiazol-2-yl)phenyl)-anthracen-10-yl)phenyl)benzo[d]thiazole, 1,4-di[4-(N,N-diphenyl)amino]styrylbenzene, 1,4-bis(4-(9H-carbazol-9-yl)styryl)benzene, (E)-6-(4-(diphenylamino)styryl)-N,N-diphenylnaphthalene-2-amine, bis(2,4-difluorophenylpyridinato)(5-(pyridin-2-yl)-1H-tetrazolate)iridium(III), bis(3-trifluoromethyl-5-(2-pyridyl)pyrazole)((2,4-difluorobenzyl)diphenylphosphinate)iridium(III), bis(3-trifluoromethyl-5-(2-pyridyl)pyrazolate)(benzyl-diphenylphosphinate)iridium(III), bis(1-(2,4-difluorobenzyl)-3-methylbenzimidazolium)(3-(trifluoromethyl)-5-(2-pyridyl)-1,2,4-triazolate)iridium(III), bis(3-trifluoromethyl-5-(2-pyridyl)pyrazolate)(4',6'-difluorophenylpyridinate)iridium(III), bis(4',6'-difluorophenylpyridinato)(3,5-bis(trifluoromethyl)-2-(2'-pyridyl)pyrolate)iridium(III), bis(4',6'-difluorophenylpyridinato)(3-(trifluoromethyl)-5-(2-pyridyl)-1,2,4-triazolate)iridium(III), (Z)-6-mesityl-N-(6-mesitylquinolin-2(1H)-ylidene)quinoline-2-amine-BF₂, (E)-2-(2-(4-(dimethylamino)styryl)-6-methyl-4H-pyran-4-ylidene)malononitrile, 4-(dicyanomethylene)-2-methyl-6-julolidyl-9-enyl-4H-pyrane, 4-(dicyanomethylene)-2-methyl-6-(1,1,7,7-tetramethyl-julolidyl-9-enyl)-4H-pyrane, 4-(dicyanomethylene)-2-t-butyl-6-(1,1,7,7-tetramethyl-julolidin-4-yl-vinyl)-4H-pyrane, tris(dibenzoylmethane)phenanthrolineuropium(III), 5,6,11,12-tetraphenylnaphthacene, bis(2-benzo[b]thiophen-2-yl-pyridine)(acetylacetonate)-iridium(III), tris(1-phenylisoquinoline)iridium(III), bis(1-phenylisoquinoline)(acetylacetonate)iridium(III), bis[1-(9,9-dimethyl-9H-fluoren-2-yl)-isoquinoline](acetylacetonate)iridium(III), bis[2-(9,9-dimethyl-9H-fluoren-2-yl)quinoline](acetylacetonate)iridium(III), tris[4,4'-di-t-butyl-(2,2')-bipyridine]ruthenium(III) • bis(hexafluorophosphate), tris(2-phenylquinoline)iridium(III), bis(2-phenylquinoline)(acetylacetonate)iridium(III), 2,8-di-t-butyl-5,11-bis(4-t-butylphenyl)-6,12-diphenyl-tetracene, bis(2-phenylbenzothiazolato)(acetylacetonate)iridium(III), 5,10,15,20-tetraphenyltetrabenzoporphyrinplatinum, osmium(II)bis(3-trifluoromethyl-5-(2-pyridine)-pyrazolate)-dimethylphenylphosphine, osmium(II)bis(3-(trifluoromethyl)-5-(4-t-butylpyridyl)-1,2,4-triazolate)diphenylmethylphosphine, osmium(II)bis(3-(trifluoromethyl)-5-(2-pyridyl)-1,2,4-triazole)dimethylphenylphosphine, osmium(II)bis(3-(trifluoromethyl)-5-(4-t-butylpyridyl)-1,2,4-triazolate)dimethylphenylphosphine, bis[2-(4-n-hexylphenyl)quinoline](acetylacetonate)-iridium(III), tris[2-(4-n-hexylphenyl)quinoline]iridium(III), tris[2-phenyl-4-methylquinoline]iridium(III), bis(2-phenylquinoline)(2-(3-methylphenyl)pyridinate)-iridium(III), bis(2-(9,9-diethyl-fluoren-2-yl)-1-phenyl-1H-benzo[d]-imidazolato)(acetylacetonate)iridium(III), bis(2-phenylpyridine)(3-pyridin-2-yl)-2H-chromen-2-onate)-iridium(III), bis(2-phenylquinoline)(2,2,6,6-tetramethylheptane-3,5-dionate)iridium(III), bis(phenylisoquinoline)(2,2,6,6-tetramethylheptane-3,5-dionate)iridium(III), iridium(III)bis(4-phenylthieno[3,2-c]pyridinate-N,C²)acetyl-acetonate, (E)-2-(2-t-butyl-6-(2-(2,6,6-trimethyl-2,4,5,6-tetrahydro-1H-pyrolo[3,2,1-ij]quinolin-8-yl)vinyl)-4H-pyran-4-ylidene)-malononitrile, bis(3-trifluoromethyl-5-(1-isoquinolyl)pyrazolate)(methyldiphenylphosphin)ruthenium, bis[(4-n-hexylphenyl)isoquinoline](acetylacetonate)-iridium(III), platinum(II)octaethylporphin, bis(2-methyldibenzo[f,h]quinoxaline)(acetylacetonate)-iridium(III), and tris[(4-n-hexylphenyl)quisoquinoline]iridium(III).

Examples of material of the electron transporting layer include 8-hydroxyquinolinolate-lithium, 2,2',2"-(1,3,5-benzinetolyl)-tris(1-phenyl-1-H-benzimidazole), 2-(4-biphenyl)5-(4-t-butylphenyl)-1,3,4-oxadiazole, 2,9-dimethyl-4,7-diphenyl-1,10-phenanthroline, 4,7-diphenyl-1,10-phenanthroline, bis(2-methyl-8-quinolinolate)-4-(phenylphenolato)aluminum, 1,3-bis[2-(2,2'-bipyridin-6-yl)-1,3,4-oxadiazo-5-yl]benzene, 6,6'-bis[5-(biphenyl-4-yl)-1,3,4-oxadiazo-2-yl]-2,2'-bipyridine, 3-(4-biphenyl)-4-phenyl-5-t-butylphenyl-1,2,4-triazole, 4-(naphthalen-1-yl)-3,5-diphenyl-4H-1,2,4-triazole, 2,9-bis(napthalen-2-yl)-4,7-diphenyl-1,10-phenanthroline, 2,7-bis[2-(2,2'-bipyridin-6-yl)-1,3,4-oxadiazo-5-yl]-9,9-dimethylfluorene, 1,3-bis[2-(4-t-butylphenyl)-1,3,4-oxadiazo-5-yl]benzene, tris(2,4,6-trimethyl-3-(pyridin-3-yl)phenyl)borane, 1-methyl-2-(4-naphthalen-2-yl)phenyl)-1H-imidazo[4,5f][1,10]-phenanthroline, 2-(naphthalen-2-yl)-4,7-diphenyl-1,10-phehanthroline, phenyl-dipyrenylphosphineoxide, 3,3',5,5'-tetra[(m-pyridyl)-phen-3-yl]biphenyl, 1,3,5-tris[(3-pyridyl)-phen-3-yl]benzene, 4,4'-bis(4,6-diphenyl-1,3,5-triazin-2-yl)biphenyl, 1,3-bis[3,5-di(pyridin-3-yl)phenyl]benzene, bis(10-hydroxybenzo[h]quinolinato)beryllium, diphenylbis(4-pyridin-3-yl)phenyl)silane, and 3,5-di(pyren-1-yl)pyridine.

The electron injection layer is formed from any of the following materials: lithium oxide (Li₂O), magnesium oxide (MgO), alumina (Al₂O₃), lithium fluoride (LiF), sodium fluoride (NaF), magnesium fluoride (MgF₂), Cesium fluoride (CsF), strontium fluoride (SrF₂), molybdenum trioxide (MoO₃), aluminum, lithium acetylacetonate (Li(acac)), lithium acetate, and lithium benzoate.

The cathode is formed from any of the following materials: aluminum, magnesium-silver alloy, aluminum lithium alloy, lithium, sodium, potassium, and cesium.

The organic EL element according to the present invention is produced in the manner exemplified below in the case where the hole injection layer is the thin film obtained from the charge transporting varnish according to the present invention.

In the above-mentioned method of producing the organic EL element, the vacuum deposition procedure for the hole transporting layer, emitting layer, electron transporting layer, and electron injection layer is replaced by the procedure by which the hole transporting layer and emitting layer are formed sequentially. The resulting organic EL element has the charge transporting thin film formed from the charge transporting varnish according to the present invention. To be concrete, the organic EL element is produced by coating the anode substrate with the charge transporting varnish according to the present invention, thereby forming the hole injection layer by the above-mentioned method, and further sequentially forming the hole transporting layer and emitting layer, and finally forming the cathode electrode by vacuum deposition.

The cathode and anode to be used are formed from the same materials as mentioned above. They may undergo cleaning and surface treatment in the same way as mentioned above.

The hole transporting layer and emitting layer are formed by dissolving or uniformly dispersing in a solvent the hole transporting polymeric material or emitting polymeric material, with or without dopant added thereto, and subsequently applying the resulting solution or suspension onto the hole injection layer or hole transporting layer, followed by baking.

Examples of the hole transporting polymeric material include
poly[(9,9-dihexylfluorenyl-2,7-diyl)-co-(N,N'-bis{p-butylphenyl}-1,4-diaminophenylene)],
poly[(9,9-dioctylfluorenyl-2,7-diyl)-co-(N,N'-bis{p-butylphenyl}-1,1'-biphenylene-4,4-diamine)],
poly[(9,9-bis{1'-penten-5'-yl}fluorenyl-2,7-diyl)-co-(N,N'-bis{p-butylphenyl}-1,4-diaminophenylene)],
poly[N,N'-bis(4-butylphenyl)-N,N'-bis(phenyl)-benzidine]-end capped with polysilsesquioxane, and
poly[(9,9-didioctylfluorenyl-2,7-diyl)-co-(4,4'-(N-(p-butylphenyl))diphenylamine)].

Examples of the light emitting polymeric material include polyfluorene derivatives, such as poly(9,9-dialkylfluorene) (PDAF), polyphenylenevinylene derivatives, such as poly(2-methoxy-5-(2'-ethylhexoxy)-1,4-phenylenevinylene) (MEH-PPV), polythiophene derivatives, such as poly(3-alkylthiophene) (PAT), and polyvinylcarbazole (PVCz).

The solvent includes toluene, xylene, and chloroform. Dissolution or uniform dispersion may be accomplished by stirring, heated stirring, or ultrasonic dispersion.

Coating may be accomplished in any way as exemplified below without specific restrictions. Ink jet printing, spraying, dipping, spin coating, transfer printing, roll coating, and brushing. Incidentally, coating should preferably be performed in an atmosphere of inert gas such as nitrogen and argon.

Baking may be accomplished by heating with an oven or hot plate in an atmosphere of inert gas or vacuum.

In the case where the charge transporting varnish according to the present invention gives rise to the thin film as the hole injection transporting layer, the organic EL element according to the present invention may be produced by the following way which is given as an example.

The anode substrate is provided with the hole injection transporting layer formed thereon, and subsequently the hole injection transporting layer is sequentially provided thereon with the emitting layer, electron transporting layer, electron injection layer, and cathode. The emitting layer, electron transporting layer, and electron injection layer are exemplified as mentioned above, and they may be produced in the same way as mentioned above.

The anode, emitting layer, emitting dopant, electron transporting layer, electron blocking layer, and cathode may be produced from the same materials as mentioned above.

An optional hole blocking layer and electron blocking layer may be interposed between the electrode and any of the above layers. The electron blocking layer may be formed from tris(phenylpyrazole)iridium.

The anode and cathode and the layers interposed between them should be formed from appropriate materials which vary depending on whether an element to be produced is of bottom emission structure or top omission structure.

The element of bottom emission structure usually has a transparent anode contiguous to the substrate, so that it emits light from the substrate side. By contrast, the element of top emission structure has a reflecting anode of metal, so that it emits light from the transparent electrode (cathode) opposite to the substrate. Consequently, regarding the anode, the element of bottom emission structure is provided with the transparent anode of ITO, and the element of top emission structure is provided with the reflecting anode of Al/Nd.

The organic EL element according to the present invention may optionally be sealed together with a water capturing agent in the usual way for protection from deterioration of characteristics.

### EXAMPLES

The present invention will be described in more detail with reference to the following Examples and Comparative Examples, which are not intended to restrict the scope of the present invention. Those apparatuses used in the Examples are listed below.
(1) ¹H-NMR measurement:
   JNM-ECP300 FT NMR SYSTEM, manufactured by JEOL Ltd.
(2) Substrate cleaning:
   Substrate cleaning equipment (reduced pressure plasma system), manufactured by Choshu Industry Co., Ltd.
(3) Coating with varnish:
   Spin coater MS-A100, manufactured by Mikasa Co., Ltd.
(4) Film thickness measurement:
   Microfigure measuring instrument SURFCORDER ET-4000, manufactured by Kosaka Laboratory Ltd.
(5) Production of EL element:
   Multi-functional deposition apparatus system C-E2L1G1-N, manufactured by Choshu Industry Co., Ltd.
(6) Measurement of luminance of EL element:
   I-V-L measuring system, manufactured by Tech World Inc.

### [1] Synthesis of compound

### [Example 1] Synthesis of triarylamine derivative A

Triarylamine derivative A represented by formula (1-31) was synthesized according to the following reaction formula:

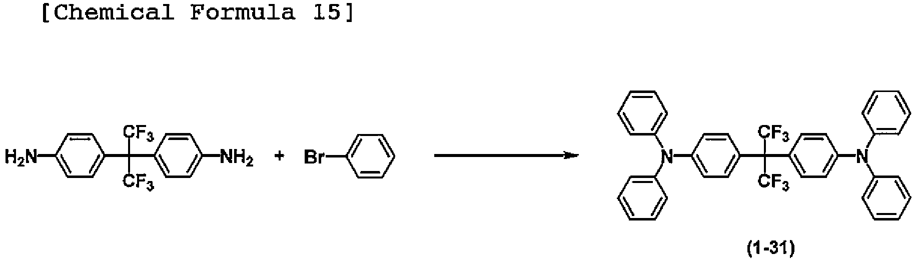

In a flask were placed 15.0 g of 4,4'-(perfluoropropane-2,2-diyl)dianiline, 0.52 g of bis(benzylideneacetone)palladium, 19.0 g of and t-butoxysodium. To the flask, with its atmosphere replaced by nitrogen, were added 260 mL of toluene, 21.0 mL of bromobenzene, and 2.60 mL of a preliminarily separately prepared toluene solution of tri-t-butylphosphine (concentration 0.143 g/mL). The contents of the flask were stirred at 50° C for two hours.

After the stirring was completed, the reaction mixture was cooled to room temperature, and the cooled mixture was then filtered. Then, liquid separation treatment was conducted using the resulting filtrate and saturated sodium chloride solution.

The thus obtained organic layer was dried with sodium sulfate. The dried organic layer was stirred at room temperature for 30 minutes together with activated carbon. With the activated carbon filtered out, the filtrate was concentrated and the resulting concentrated liquid was added dropwise to methanol (1.3 L) with stirring. Stirring was continued for one hour.

Subsequently, the resulting slurry was filtered and the residue was dried under reduced pressure. Thus, there was obtained triarylamine derivative A (yields: 27.0 g). The ¹H-NMR measurement results are shown below.
¹H-NMR (CDCl₃):
δ 7.27 to 7.30 (m, 7H), 7.20 to 7.22 (m, 5H), 7.03 to 7.13 (m, 12H), 6.97 (d, J = 9.2 Hz, 4H).

### [2] Preparation of charge transporting varnish

### [Example 2-1]

In 8 g of 1,3-dimethyl-2-imidazolidinone were dissolved, in a nitrogen atmosphere, 0.106 g of an oligoaniline derivative represented by formula (f), which is a charge transporting substance, and 0.636 g of phosphotungstic acid, which is a dopant. To the resulting solution were added 12 g of cyclohexanol and 4 g of propylene glycol, with stirring. To the resulting solution was further added 0.074 g of triarylamine derivative A, with stirring. Thus, there was obtained the charge transporting varnish. Incidentally, the oligoaniline derivative represented by formula (f) was synthesized according to the method disclosed in WO 2013/084664:

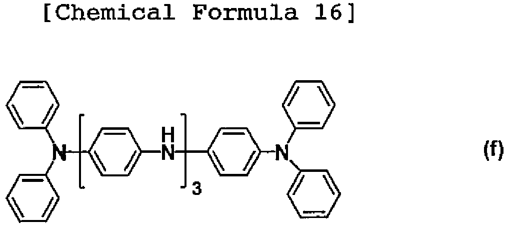

### [Comparative Example 1]

The same procedure as in Example 2-1 was repeated to prepare charge transporting varnish, except that triarylamine derivative A was not added.

### [3] Production of double-layered element and characteristic evaluation

### [Example 3-1]

The varnish obtained in Example 2-1 was applied onto an ITO substrate by spin coating, followed by drying at 50° C for five minutes and baking at 230°C for 15 minutes in the atmospheric air. Thus, the ITO substrate was coated with a 30 nm-thick uniform thin film. As the ITO substrate, a glass substrate measuring 25 mm × 25 mm × 0.7 thick formed thereon with a pattern of a 150 nm-thick film of indium tin oxide (ITO) was used, and impurities on the surface thereof were removed by an O₂ plasma cleaning apparatus (150 W, 30 seconds) before use.

Subsequently, the ITO substrate having the thin film formed thereon was coated sequentially with two layers of thin film of α-NPD and aluminum by a vapor deposition system (vacuum degree 1.0×10⁻⁵ Pa). Thus, there was obtained the double-layered element as desired. The vapor deposition for both α-NPD and aluminum was carried out at a rate of 0.2 nm/second, with their thicknesses each controlled to 30 nm and 100 nm.

The double-layered element was sealed with sealing substrates for protection from characteristic deterioration due to oxygen and water in the air. After that, it had its characteristics evaluated. Incidentally, sealing was carried out in the following way.

First, the organic EL element was held between sealing substrates in a nitrogen atmosphere with an oxygen concentration of up to 2 ppm and a dew point of up to -85° C. Second, they were adhered to each other with an adhesive (Moresco Moisture Cut WB90US(P), manufactured by MORESCO Corporation). In this instance, a water capturing agent (HD-071010W-40, manufactured by Dynic Corporation) was placed inside the sealing substrates together with the organic EL element. After the thus adhered sealing substrates were irradiated with UV light (wavelength: 365 nm, irradiation amount: 6,000 mJ/cm²), an annealing treatment was conducted at 80° C for one hour, to cure the adhesive.

### [Comparative Example 2]

The same procedure as in Example 3-1 was repeated to prepare the double-layered element except that the varnish obtained in Example 2-1 was replaced by the varnish obtained in Comparative Example 1.

The above-mentioned elements were examined for current density at a drive voltage of 3V. The results are shown in Table 19.

**[Table 19]**

| | Current density (mA/cm²) |
|---|---|
| Example 3-1 | 1,590 |
| Comparative Example 2 | 1,210 |

The results shown in Table 19 suggest that it is possible to achieve the improved hole transportability to the hole transporting layer if the charge transporting varnish is incorporated with the triarylamine derivative according to the present invention.

### [4] Production of organic EL element and characteristic evaluation

### [Example 4-1]

The varnish obtained in Example 2-1 was applied onto an ITO substrate by spin coating, followed by drying at 50° C for five minutes and baking at 230° C for 15 minutes in the atmospheric air. Thus, there was obtained a 30 nm-thick uniform thin film formed on the ITO substrate. As the ITO substrate, a glass substrate measuring 25 mm × 25 mm × 0.7 thick formed thereon with a pattern of a 150 nm-thick film of indium tin oxide (ITO) was used, and impurities on the surface thereof were removed by an O₂ plasma cleaning apparatus (150 W, 30 seconds) before use.

Subsequently, the ITO substrate having a thin film formed thereon was coated sequentially with four layers of thin film of α-NPD, Alq₃, lithium fluoride, and aluminum by a vapor deposition system (vacuum degree 1.0×10⁻⁵ Pa). Thus, there was obtained the organic EL element as desired. The vapor deposition was carried out at a rate of 0.2 nm/second for α-NPD, Alq₃, and aluminum and 0.02 nm/second for lithium fluoride, with their thicknesses each controlled to 30 nm, 40 nm, 0.5 nm, and 1,000 nm.

Incidentally, the EL element was sealed for protection from characteristic deterioration due to oxygen and water in the air by the same way as in Example 3-1.

### [Comparative Example 3]

The same procedure as in Example 4-1 was repeated to prepare the organic EL element except that the varnish obtained in Example 2-1 was replaced by the varnish obtained in Comparative Example 1.

The produced elements were examined for current density, luminance, and current efficiency at a drive voltage of 5 V. The results are shown in Table 20. Incidentally, each element has an emitting area of 2 mm × 2 mm.

**[Table 20]**

| | Current density (mA/cm²) | Luminance (cd/m²) | Current efficiency (cd/A) |
|---|---|---|---|
| Example 4-1 | 230 | 5,660 | 2.5 |
| Comparative Example 3 | 200 | 4,860 | 2.5 |

The results shown in Table 20 suggest that it is possible to obtain the organic EL element with outstanding luminance characteristics if the hole injection layer is the thin film formed from the charge transporting varnish including the triarylamine derivative according to the present invention.

## Claims

1. A triarylamine derivative represented by formula (1): 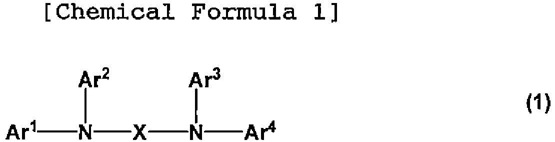 [wherein, Ar¹ to Ar⁴ each independently represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group; and X represents a divalent group represented by formula (2): 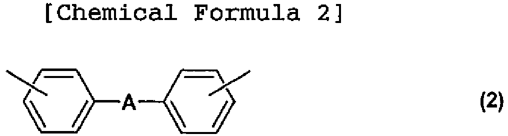 (wherein A represents a fluoroalkanediyl group having 1 to 6 carbon atoms)].

2. The triarylamine derivative of claim 1, wherein Ar¹ to Ar⁴ simultaneously represent a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, or 9-phenanthryl group.

3. The triarylamine derivative of claim 1 or 2, wherein X represents any group represented by formulae (X1) to (X3): 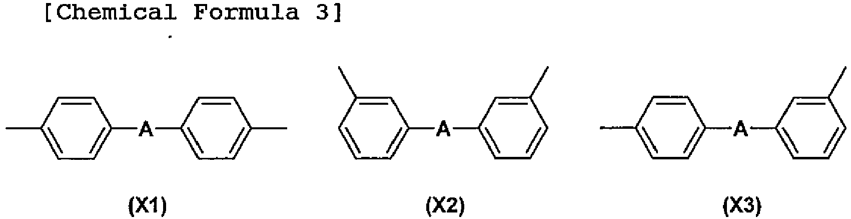 (wherein A is defined as above).

4. The triarylamine derivative of claim 3, wherein X represents a group represented by formula (X1).

5. The triarylamine derivative of any one of claims 1 to 4, wherein A represents a perfluoromethanediyl group, perfluoroethane-1,2-diyl group, perfluoropropane-1,3-diyl group, perfluoropropane-2,2-diyl group, perfluorobutane-1,4-diyl group, perfluoropentane-1,5-diyl group, or perfluorohexane-1,6-diyl group.

6. The triarylamine derivative of claim 5, wherein A represents a perfluoropropane-2,2-diyl group.

7. A charge transporting varnish comprising:
the triarylamine derivative of any one of claims 1 to 6;
a charge transporting substance; and
an organic solvent.

8. The charge transporting varnish of claim 7, further comprising a dopant.

9. The charge transporting varnish of claim 8, wherein the dopant includes heteropoly acid.

10. A charge transporting thin film produced by use of the charge transporting varnish of any one of claims 7 to 9.

11. An organic electroluminescence element comprising the charge transporting thin film of claim 10.

12. A method of producing a charge transporting thin film, the method comprising:
coating a substrate with the charge transporting varnish of any one of claims 7 to 9; and
evaporating a solvent.

13. A method of producing the triarylamine derivative of claim 1, the method comprising
reacting in the presence of catalyst an amine compound represented by formula (3) with compounds represented by formulae (4) to (7): 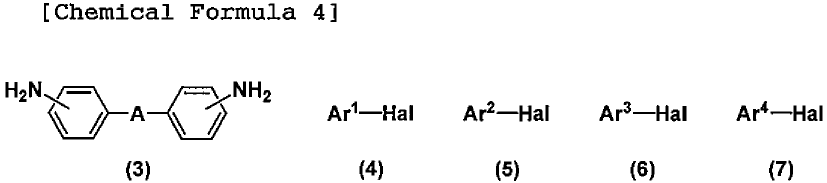 (wherein Ar¹ to Ar⁴ and A are defined as above and Hal represents a halogen atom or pseudohalogen group).

14. A method of producing the triarylamine derivative of claim 1, the method comprising
reacting in the presence of catalyst a compound represented by formula (3') with amine compounds represented by formulae (4') and (5'): 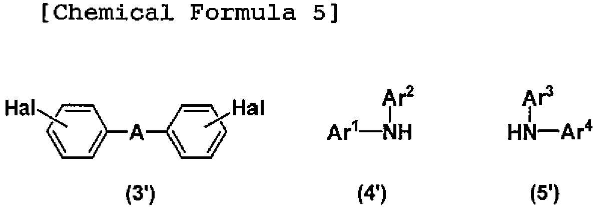 (wherein Ar¹ to Ar⁴ and A are defined as above and Hal represents a halogen atom or pseudohalogen group).
